# EUROPEAN PATENT APPLICATION

(11) **EP 3 480 775 A1**
(43) Date of publication of application: **08.05.2019**
(21) Application number: 17819828.9
(22) Date of filing: 12.06.2017
(51) Int. Cl.: G06Q 50/22, A61G 12/00

(54) **CENTRAL PROCESSING DEVICE AND CENTRAL PROCESSING METHOD FOR MONITORED-PERSON MONITORING SYSTEM, AND MONITORED-PERSON MONITORING SYSTEM**

(30) Priority: 29.06.2016 JP 2016128580
(71) Applicant: Konica Minolta, Inc., Tokyo 100-7015 (JP)
(72) Inventor: NISHIKADO, Masashi, Tokyo 100-7015 (JP); KOGO, Shoji, Tokyo 100-7015 (JP)
(74) Representative: Gille Hrabal
(86) International application number: PCT/JP2017/021566
(87) International publication number: WO 2018/003463

(57) **Abstract**

A central processing device and a central processing method according to the present invention performs/includes: storing a record format that is created in accordance with a type of nursing care, and is designed for inputting details of nursing care as a nursing care record; and, when receiving a type notifying communication signal for issuing a notification of a type of nursing care in a nursing care record from a terminal device, returning a format notifying communication signal containing the record format corresponding to the type of nursing care contained in the type notifying communication signal, to enable the terminal device to input the details of the nursing care for each of the monitored persons while changing the monitored persons. A monitored-person monitoring system according to the present invention includes the central processing device.

## Description

### Technical Field

The present invention relates to a central processing device and a central processing method for a monitored-person monitoring system that monitors, with devices, a monitored person who is a subject to be monitored, and to the monitored-person monitoring system.

### Background Art

Because of the living standards, the hygienic environments, the medical standards, and the like that were improved by the rapid economic growth after the Second World War, our country (Japan) has become an aging society, or more accurately, a super-aging society in which the percentage of the elderly population or the proportion of the population aged 65 or older to the total population is higher than 21%. According to the statistics as of September 2013, the elderly population is approximately 31.86 million, and its aging rate is 25.0%, with one out of every four being an elderly person. Some estimates show that the elderly population will reach 37.41 million in 2035, and one out of every three will be an elderly person (Japan Bureau of Statistics, the Ministry of Internal Affairs and Communications). In such an aging society, the people in need of nursing and the people in need of care (the people in need of nursing care) due to illness, injury, aging, and the like are likely to increase at a higher rate than those in need of nursing care in a normal society that is not an aging society. Our country is also a society with fewer children, and the total birthrate in 2013 was 1.43, for example. For this reason, there are cases where elderly family members (spouses, children, and siblings) take care of other elderly family members in need of nursing care.

Those in need of nursing care go to hospitals or facilities such as welfare facilities for the aged (short-term facilities for the elderly, nursing homes for the elderly, and intensive care homes for the elderly under the Japanese laws and regulations), and receive treatment and care at those facilities. At such facilities, those in need of nursing care sometimes have situations. For example, a person in need of nursing care is injured after falling off the bed or falling while walking, or gets out of bed and wanders around. To counter such situations, it is necessary to take appropriate measures as soon as possible. Further, if such situations are not solved, even more serious situations might be caused. Therefore, at the facilities, nurses and caregivers (nursing care staff) regularly inspect those in need of nursing care, to confirm their safety and conditions. The nurses, the caregivers, and the like record the results of the inspections as nursing records and care records (nursing care records).

However, the increase in the number of nurses and the increase in the number of caregivers are smaller than the increase in the number of people in need of nursing care, and therefore, there is a chronic understaffing problem in the nursing care business. Furthermore, the number of nursing care workers on the evening shift and the night shift is smaller than that during the daytime. Therefore, the workload per person is larger during the nighttime, and there is a demand for a reduction in the workload. The situations of elderly people taking care of other elderly people are also seen at those facilities, and it is not rare that elderly nursing care workers take care of elderly patients in need of nursing care. However, people normally lose physical strength with age, and the same workload puts greater burdens on healthy but aged care workers than on younger ones. Elderly care workers are also slower in action and judgment.

To alleviate such worker shortages and the burdens on the nursing care workers, there is a demand for a technology of supplementing nursing and caregiving work. For this reason, studies and development are being actively made these days in the subject monitoring technologies for monitoring persons who are in need of nursing care and are subjects to be monitored (monitored persons).

Patent Literature 1 suggests a nurse call system as an example of such technologies. In the nurse call system disclosed in Patent Literature 1, a nursed person is wearing a wristband equipped with a wireless tag storing the nursed person identification ID. In a case where recording is performed on an electronic medical record, a medical/nursing worker carries a mobile information terminal, and brings the mobile information terminal close to the wristband of the nursed person, to read the nursed person identification ID from the wristband. The care worker then selects treatment details from a list of selectable treatment details displayed on the mobile information terminal. As a result, the medical/nursing worker identification ID, the treatment details, and the nursed person identification ID are transmitted from the mobile information terminal to a controller, and information indicating that the medical/nursing worker having the medical/nursing worker identification ID has performed the treatment shown in the specific treatment on the nursed person having the nursed person identification ID is recorded, together with the data reception time, into the electronic medical record stored in a controller storage unit of the controller (see paragraphs [0157] through [0166] in Patent Literature 1, for example).

To identify a subject in nursing or caregiving as required for nursing care recording in the nurse call system disclosed in Patent Literature 1, a medical/nursing worker reads the nursed person identification ID from the wristband of a nursed person by bringing his/her mobile information terminal close to the wristband of the nursed person, and then selects treatment details from the list of selectable treatment details displayed on the mobile information terminal. Therefore, even in a case where nursing care recording is performed while the same treatment details are performed on different subjects, the medical/nursing worker needs to repeatedly read a nursed person identification ID and select treatment details, taking trouble and consuming a long time.

### Citation List

### Patent Literature

Patent Literature 1: JP 2014-87524 A

### Summary of Invention

The present invention has been made in view of the above circumstances, and aims to provide a central processing device and a central processing method for a monitored-person monitoring system capable of further reducing the effort and time required for performing nursing care recording, and to provide the monitored-person monitoring system.

A central processing device and a central processing method according to the present invention performs/includes: storing a record format that is created in accordance with a type of nursing care, and is designed for inputting details of nursing care as a nursing care record; and, when receiving a type notifying communication signal for issuing a notification of a type of nursing care in a nursing care record from a terminal device, returning a format notifying communication signal containing the record format corresponding to the type of nursing care contained in the type notifying communication signal, to enable the terminal device to input the details of the nursing care for each of the monitored persons while changing the monitored persons. A monitored-person monitoring system according to the present invention includes the central processing device.

These and other objects, features and advantages of the present invention will become apparent from the following detailed description and the accompanying drawings.

### Brief Description of Drawings

Fig. 1 is a diagram showing the configuration of a monitored-person monitoring system according to an embodiment.
Fig. 2 is a diagram showing the configuration of a management server device in the monitored-person monitoring system.
Fig. 3 is a diagram showing the structure of a monitoring information table stored in the management server device.
Fig. 4 is a diagram showing the structure of an inter-device information table stored in the management server device.
Fig. 5 is a diagram showing the structure of a sensor information table stored in the management server device.
Fig. 6 is a front view of the exterior of a mobile terminal device in the monitored-person monitoring system.
Fig. 7 is a diagram showing the configuration of a mobile terminal device in the monitored-person monitoring system.
Fig. 8 is a diagram showing an example of a standby screen displayed on the mobile terminal device.
Fig. 9 is a diagram showing an example of a monitoring information screen displayed on the mobile terminal device.
Fig. 10 is a diagram showing an example of a nurse call receiving screen displayed on the mobile terminal device.
Fig. 11 is a sequence diagram showing a nursing care recording operation in a first mode in the monitored-person monitoring system.
Fig. 12 is a sequence diagram showing a nursing care recording operation in a second mode in the monitored-person monitoring system.
Fig. 13 is a diagram showing an example of a subject selection screen displayed on the mobile terminal device.
Fig. 14 is a diagram showing an example of a submenu screen displayed on the mobile terminal device.
Fig. 15 is a diagram showing an example of a first format selection screen displayed on the mobile terminal device.
Fig. 16 is a diagram showing an example of a food care record format screen displayed on the mobile terminal device.
Fig. 17 is a diagram showing an example of a morning care record format screen (an evening care record format screen) displayed on the mobile terminal device.
Fig. 18 is a diagram showing an example of a toilet support record format screen displayed on the mobile terminal device.
Fig. 19 is a diagram showing the subject selection screen after monitored persons are selected on the subject selection screen displayed on the mobile terminal device.
Fig. 20 is a diagram showing an example of a second format selection screen displayed on the mobile terminal device.
Fig. 21 is a diagram for explaining the procedures for inputting nursing care records in the second mode.

### Description of Embodiments

The following is a description of an embodiment of the present invention, with reference to the drawings. It is to be noted that, in the respective drawings, components denoted by the same reference numerals are the same components, and explanation of them will not be unnecessarily repeated. In this specification, like components are denoted by a reference numeral without any suffix when being collectively referred to, and like components are denoted by reference numerals with suffixes when being individually referred to.

A monitored-person monitoring system according to an embodiment monitors a monitored person (the subject to be watched) Ob who is a monitoring target (the watching target) to be monitored (to be watched), and includes: a sensor device that is provided for the monitored person Ob, and detects a predetermined event preset beforehand with respect to the monitored person Ob; a central processing device that is communicably connected to the sensor device, and manages events detected by the sensor device and received from the sensor device; and a terminal device that is communicably connected to the central processing device, and receives a notification of an event detected by the sensor device via the central processing device. The central processing device includes: a format information storage unit that stores a record format prepared in accordance with the type of nursing care including nursing and/or caregiving, the record format being designed for inputting the details of the nursing care as a nursing care record; and a nursing care record processing unit that extracts the record format corresponding to the type of the nursing care contained in a type notifying communication signal from the format information storage unit, and returns a format notifying communication signal containing the extracted record format to the terminal device as the transmission source that has transmitted the type notifying communication signal, to enable the terminal device to input the details of nursing care for respective monitored persons while changing the monitored persons, in a case where the type notifying communication signal for issuing a notification of the type of nursing care in a nursing care record to be input for each of the monitored persons has been received from the terminal device. The terminal device may be a type of device, but, in this embodiment, the terminal device is two kinds of devices; a fixed terminal device and mobile terminal devices. The principal difference between the fixed terminal device and the mobile terminal devices is that the fixed terminal device is operated in a fixed manner while the mobile terminal devices are carried and operated by the monitoring persons (users) such as nurses, caregivers, or the like, for example. Other than that, the fixed terminal device and the mobile terminal devices are substantially the same. Such a monitored-person monitoring system will be described below in greater detail.

First, the configuration of a monitored-person monitoring system according to this embodiment is described.

### (Configuration)

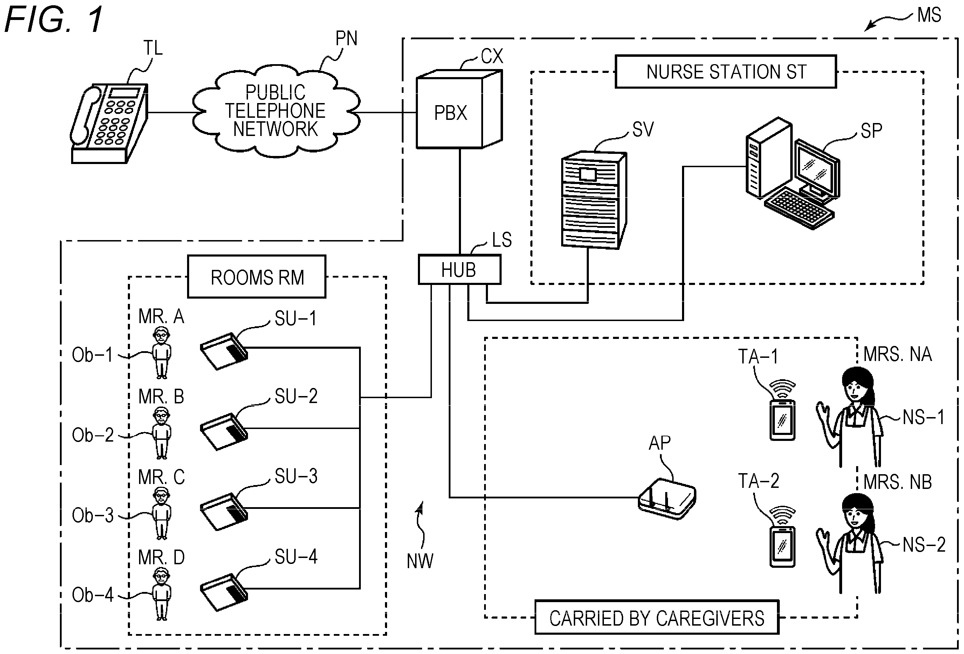

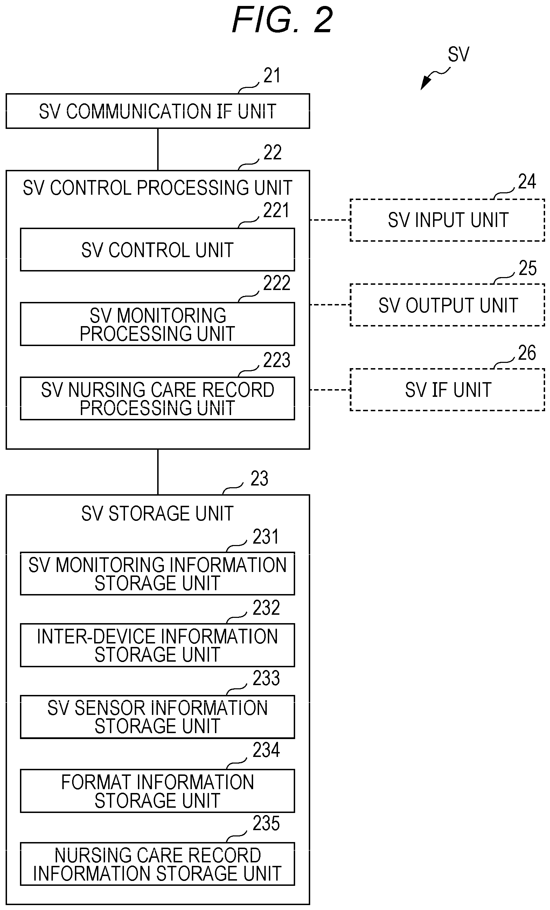

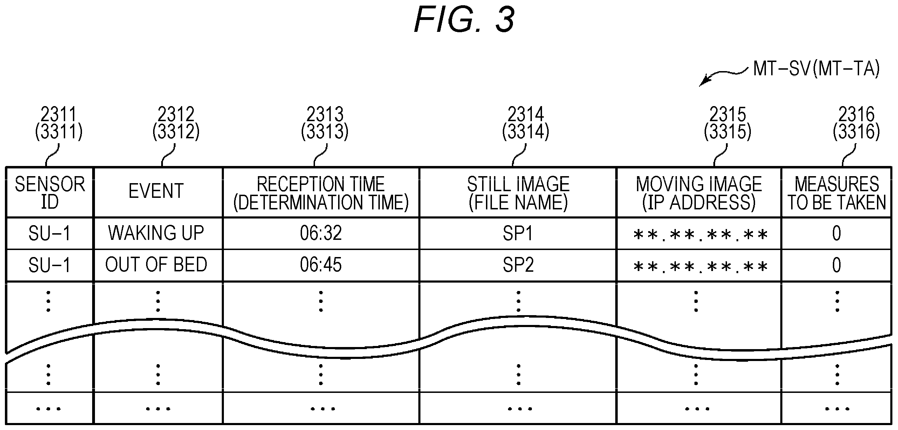

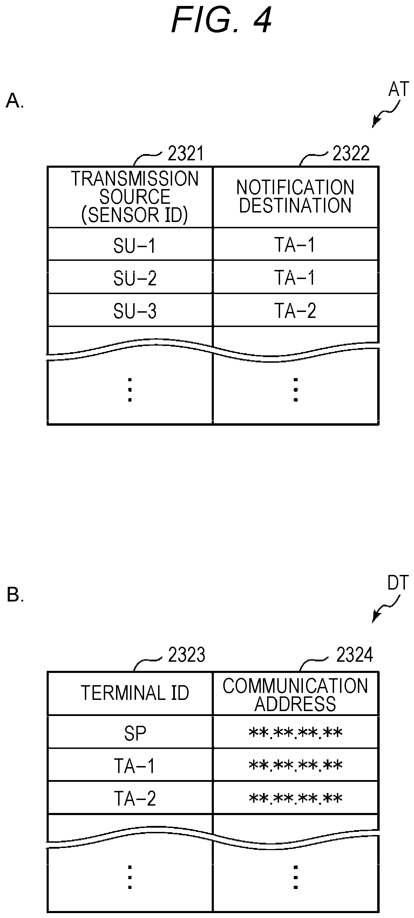

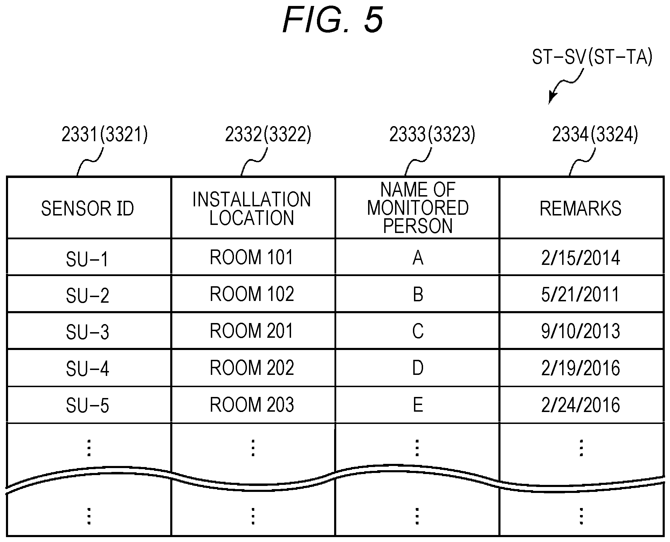

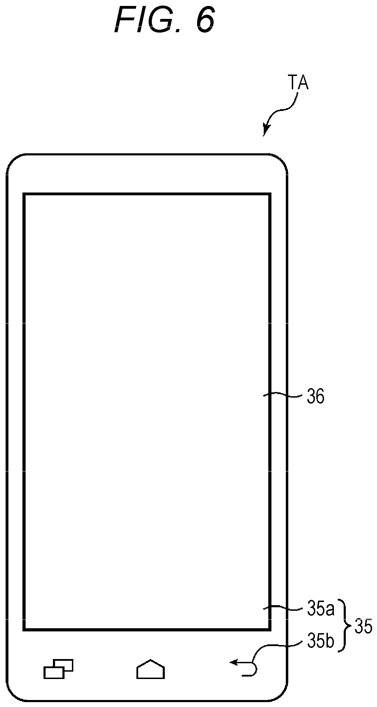

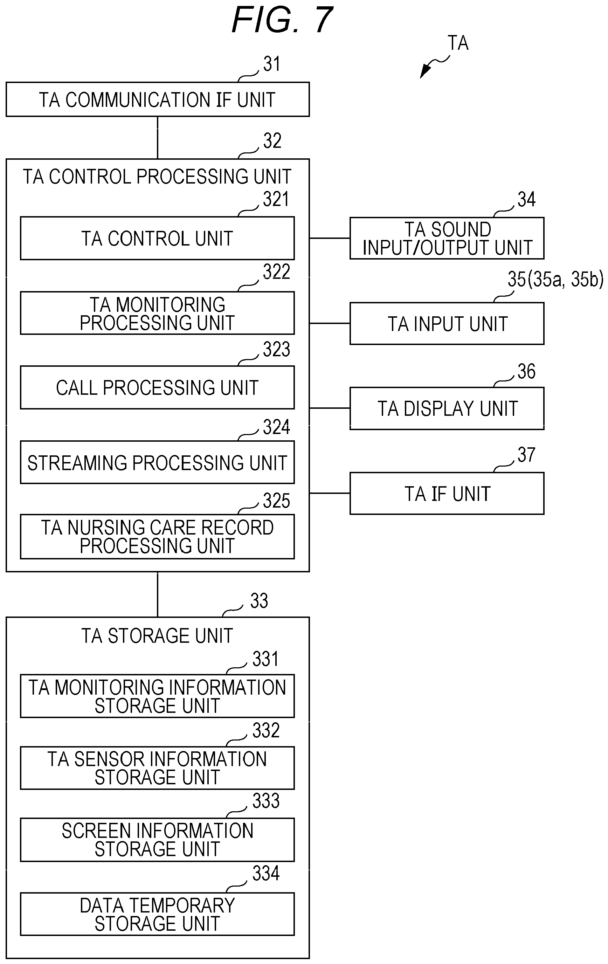

Fig. 1 is a diagram showing the configuration of a monitored-person monitoring system according to the embodiment. Fig. 2 is a diagram showing the configuration of a management server device in the monitored-person monitoring system according to the embodiment. Fig. 3 is a diagram showing the structure of a monitoring information table stored in the management server device. Fig. 4 is a diagram showing the structure of an inter-device information table stored in the management server device. Fig. 5 is a diagram showing the structure of a sensor information table stored in the management server device. Fig. 6 is a front view of the exterior of a mobile terminal device in the monitored-person monitoring system according to the embodiment. Fig. 7 is a diagram showing the configuration of a mobile terminal device in the monitored-person monitoring system according to the embodiment.

More specifically, as shown in Fig. 1, a monitored-person monitoring system MS includes one or more sensor devices SU (SU-1 through SU-4), a management server device SV, a fixed terminal device SP, one or more mobile terminal devices TA (TA-1 and TA-2), and a private branch exchange (PBX) CX, for example. These devices are communicably connected in a wired or wireless manner via network (or a communication line) NW such as a local area network (LAN). The network NW may be provided with a relay device that relays communication signals, such as a repeater, a bridge, or a router, for example. In the example shown in Fig. 1, the sensor devices SU-1 through SU-4, the management server device SV, the fixed terminal device SP, the mobile terminal devices TA-1 and TA-2, and the private branch exchange CX are communicably connected to one another by the LAN (a LAN compliant with the IEEE 802.11 standard, for example) that includes an L2 switch hub LS and an access point AP, and are compatible with both cable and wireless communications. More specifically, the sensor devices SU-1 through SU-4, the management server device SV, the fixed terminal device SP and the private branch exchange CX are connected to the hub LS, and the mobile terminal devices TA-1 and TA-2 are connected to the hub LS via the access point AP. As Internet protocols such as Transmission Control Protocol (TCP) and Internet Protocol (IP) are used, the network NW forms a so-called intranet.

The private branch exchange (line switcher) CX is connected to the network NW, and performs internal calls between the mobile terminal devices TA by controlling internal calls such as outgoing calls, incoming calls, and communications between the mobile terminal devices TA. The private branch exchange CX is connected to an external telephone set TL such as a fixed telephone set or a mobile telephone via a public telephone network PN such as a land line network or a mobile telephone network, to perform external calls between the external telephone set TL and the mobile terminal devices TA by controlling external calls such as outgoing calls, incoming calls, and communications between the external telephone set TL and the mobile terminal devices TA, for example. The private branch exchange CX is a digital exchange, an Internet Protocol private branch exchange (IP-PBX), or the like, for example.

The monitored-person monitoring system MS is disposed at an appropriate place for a monitored person Ob. A monitored person (a watching subject) Ob is a person who needs nursing due to sickness or injury, a person who needs care due to a decline in physical ability, a person who lives along, or the like, for example. Particularly, from the viewpoint of early detection and early treatment, a monitored person Ob is preferably a person who needs to be discovered when a predetermined unfavorable event such as an abnormal condition occurs to the person, for example. Therefore, the monitored-person monitoring system MS is suitably installed in a building such as a hospital, a welfare facility for the aged, or a residence, depending on the type of the monitored person Ob. In the example shown in Fig. 1, the monitored-person monitoring system MS is installed in a building of a nursing home that includes rooms such as rooms RM occupied by monitored persons Ob, and a nurse station.

Each sensor device SU is a device that has a communication function or the like for communicating with other devices SV, SP, and TA via the network NW, detects a predetermined event related to the monitored person Ob, and notifies the management server device SV of the event. The predetermined event is preferably a predetermined event that needs to be dealt with. For example, in this embodiment, the predetermined event is a preset predetermined action and a nurse call (NC) made by the monitored person Ob. More specifically, each sensor device SU includes: a communication interface circuit (a LAN card or the like, for example) for communicating with the other devices SV, SP, and TA via the network NW; an image sensor (a visible camera, a near-infrared camera, or the like, for example) that images the monitored person Ob from above (preferably from right above (from the ceiling, for example)), and generates an image; a behavior detection processing circuit that detects a preset predetermined action of the monitored person Ob as an example of the predetermined event in accordance with an output (the image) from the image sensor, and notifies the management server device SV of the action; a nurse call processing circuit that accepts a nurse call as another example of the predetermined event, and notifies the management server device SV of the nurse call; a call processing circuit that makes a voice call with a terminal device SP, TA, or the like; an image transmission processing circuit that transmits an output (an image (including a still image and a moving image)) from the image sensor to another predetermined device SV, SP, or TA; a control circuit that controls the above circuits; and peripheral circuits of the above circuits, such as memory elements, for example.

The predetermined action may be one of the four actions: the monitored person Ob going to bed, the monitored person Ob waking up, the monitored person Ob getting out of bed, and the monitored person Ob falling, for example. Note that the fall may include the monitored person Ob falling out of bed, or, separately from the fall, the predetermined action may include a fall. For example, the behavior detection processing circuit detects the head of the monitored person Ob from an image (a target image) captured by the image sensor, detects the monitored person Ob waking up and falling in accordance with a temporal change in the size of the detected head of the monitored person Ob, detects the monitored person Ob from the target image captured by the image sensor, and detects the monitored person Ob getting out of bed and going to bed in accordance with an overlap (the size of the overlapping region) between the detected monitored person Ob and the bedding. More specifically, first, the existence region of the bed BD, a first threshold value Th1, and a second threshold value Th2 are stored. The first threshold value Th1 is a value for identifying the size of the head of a person in a lying posture and the size of the head of a person in a seated posture within the existence region of the bed BD. The second threshold value Th1 is a value for determining whether the size of the head is that of a person in a lying posture in the rooms RM excluding the existence region of the bed BD. The behavior detection processing circuit extracts a moving object region as the region of the person of the monitored person Ob from the target image by a background differencing technique or a frame differencing technique, for example. The behavior detection processing circuit next extracts, from the moving object region, the head region of the monitored person Ob by circular or elliptical Hough transform, by pattern matching using a model of a head prepared in advance, or by neural network learned for head detection, for example. The behavior detection processing circuit then detects rising or falling depending on the extracted position and size of the head. For example, in a case where the extracted head position is within the existence region of the bed BD, and the extracted head size has temporally changed from the size in a lying posture to the size in a seated posture in accordance with the first threshold value Th1, the behavior detection processing circuit determines that the person has woke up, and detects the waking up. For example, in a case where the extracted head position is within the rooms RM excluding the existence region of the bed BD, and the extracted head size has temporally changed from a certain size to the size in a lying posture in accordance with the second threshold value Th2, the behavior detection processing circuit determines that the person has fallen, and detects the fall. The behavior detection processing circuit then detects an overlapping region in which the moving object region extracted from the target image as described above and the existence region of the bed BD overlap with each other, and detects the person getting out of bed or going to bed, depending on a temporal change in the overlapping region. For example, in a case where the overlapping region temporally changes from a state in which there is the detected overlapping region to a state in which the overlapping region no longer exists, the behavior detection processing circuit determines that the person has got out of bed, and detects the getting out of bed. For example, in a case where there is a temporal change from a state in which the detected overlapping region does not exist to a state in which the entire overlapping region overlaps with the existence region of the bed BD (or a state in which the moving object region is completely included in the existence region of the bed BD) through detection of the existence of the overlapping region, the behavior detection processing circuit determines that the person has gone to bed, and detects the going to bed.

The management server device SV is notified of the predetermined event by the sensor device SU through a first event notifying communication signal. This first event notifying communication signal contains the sensor ID of the device and event information indicating the details of the event. The sensor ID (sensor identifier) is the identifier for specifying and identifying the sensor device SU. In this embodiment, the event information indicates one or more of the following events: going to bed, waking up, getting out of bed, a fall, and a nurse call. The first event notifying communication signal may contain an image captured by the image sensor. Particularly, in a case where the event information indicates going to bed, waking up, getting out of bed, or a fall, the first event notifying communication signal preferably contains the image used in detecting the predetermined action (or the last image in a case where the detection is performed with the use of more than one image, for example). The image may be a still image and/or a moving image. In this embodiment, a still image is first sent, and a moving image is then delivered in response to a request from the user, as will be described later. It should be noted that a moving image may be delivered first. Alternatively, a still image and a moving image may be transmitted, and the sill image and the moving image may be displayed on divided screens on a terminal device SP or TA.

In Fig. 1, four sensor devices that are first through fourth sensor devices SU-1 through SU-4 are shown as an example. The first sensor device SU-1 is installed in the room RM-1 (not shown) of Mr. A Ob-1, who is one of the monitored persons Ob, the second sensor device SU-2 is installed in the room RM-2 (not shown) of Mr. B Ob-2, who is one of the monitored persons Ob, the third sensor device SU-3 is installed in the room RM-3 (not shown) of Mr. C Ob-3, who is one of the monitored persons Ob, and the fourth sensor device SU-4 is installed in the room RM-4 (not shown) of Mr. D Ob-4, who is one of the monitored persons Ob.

The management server device SV has a communication function for communicating with the other devices SU, TA, and SP via the network NW. When receiving a notification of the predetermined event from a sensor device SU, the management server device SV manages information about monitoring of the monitored person Ob (the information about monitoring is monitoring information (in this embodiment, for example, the predetermined event (such as the type of a predetermined action detected by the sensor device SU and a nurse call received by the sensor device SU), an image of the monitored person Ob (a still image and a moving image), the time of reception of the notification, and the like)), notifies a predetermined terminal device SP or TA of the predetermined event (by re-notification re-reporting, or transmission), and provides a client (a terminal device SP, TA, or the like in this embodiment) with data in response to a request from the client. In this manner, the management server device SV (an example of the central processing device) manages the entire monitored-person monitoring system MS. In this embodiment, the management server device SV performs predetermined information processing concerning nursing care records that are records of details of nursing care including nursing and/or caregiving. As shown in Fig. 2, this management server device SV includes a server-side communication interface unit (a SV communication IF unit) 21, a server-side control processing unit (a SV control processing unit) 22, and a server-side storage unit (a SV storage unit) 23, for example.

The SV communication IF unit 21 is a communication circuit that is connected to the SV control processing unit 22, and is designed for performing communication under the control of the SV control processing unit 22. The SV communication IF unit 21 generates a communication signal containing the data that has been input from the SV control processing unit 22 and is to be transferred, in accordance with the communication protocol being used in the network NW of the monitored-person monitoring system MS. The SV communication IF unit 21 then transmits the generated communication signal to the other devices SV, SP, and TA via the network NW. The SV communication IF unit 21 receives a communication signal from another device SV, SP, or TA via the network NW, extracts data from the received communication signal, and outputs the extracted data to the SV control processing unit 22 after converting the extracted data into data in a format that can be processed by the SV control processing unit 22. The SV communication IF unit 21 includes a communication interface circuit compliant with the IEEE 802.11 standard or the like, for example.

The SV storage unit 23 is a circuit that is connected to the SV control processing unit 22 and stores various kinds of predetermined programs and various kinds of predetermined data, under the control of the SV control processing unit 22. The various kinds of predetermined programs include control processing programs, such as an SV control program for controlling the respective components of the management server device SV in accordance with the functions of the respective components, an SV monitoring processing program for performing predetermined information processing relating to monitoring of the monitored persons Ob, and a SV nursing care record processing program for performing predetermined information processing concerning nursing care records that are records of details of nursing care including nursing and/or caregiving, for example. The various kinds of predetermined data include the data necessary in executing the respective programs, such as a server identifier (server ID) that is an identifier for specifying the management server device SV and identifying the management server device SV of the system, the above mentioned monitoring information about the monitored persons Ob, inter-device information indicating information relating to interactions between the devices SU, SP, and TA such as the notification destination of the predetermined event, sensor information concerning the sensor devices SU, format information concerning the record format, and nursing care record information relating to nursing care records. The SV storage unit 23 includes a read only memory (ROM) that is a nonvolatile memory element, an electrically erasable programmable read only memory (EEPROM) that is a rewritable nonvolatile memory element, and the like, for example. The SV storage unit 23 includes a random access memory (RAM) or the like that serves as a working memory for the SV control processing unit 22 and stores data and the like generated during execution of the predetermined programs. To store the monitoring information, the inter-device information, the sensor information, the format information, and the nursing care record information, the SV storage unit 23 functionally includes a server-side monitoring information storage unit (a SV monitoring information storage unit) 231, an inter-device information storage unit 232, a server-side sensor information storage unit (a SV sensor information storage unit) 233, a format information storage unit 234, and a nursing care record information storage unit 235, respectively.

The SV monitoring information storage unit 231 stores the monitoring information about the monitored person Ob transmitted to and received from each of the devices SU, SP, and TA. More specifically, in this embodiment, the SV monitoring information storage unit 231 stores the monitoring information, which is the sensor ID, the event information (going to bed, waking up, getting out of bed, a fall, or a nurse call in this embodiment), the reception time, the target image (a still image and a moving image), and presence/absence of measures to be taken, in accordance with the respective pieces of information contained in a communication signal such as the first event notifying communication signal. These stored items are associated with one another.

In this embodiment, this monitoring information is stored in a table format in the SV monitoring information storage unit 231. For example, as shown in Fig. 3, the server-side monitoring information table (SV monitoring information table) MT-SV that registers this monitoring information includes: a sensor ID field 2311 that registers the sensor IDs contained in communication signals received from the respective devices SU, SP, and TA; an event field 2312 that registers the event information contained in the received communication signals; a reception time field 2313 that registers the reception times of the received communication signals; a still image field 2314 that registers the still images contained in the received communication signals; a moving image field 2315 that registers the communication addresses (such as IP addresses, for example) of the sensor devices SU corresponding to the sensor IDs contained in the received communication signals; a measure field 2316 that registers presence/absence of acceptance of measures to be taken to cope with the event information contained in the received communication signals. The server-side monitoring information table MT-SV also includes records for the respective received communication signals (the respective events). The still image field 2314 may register the image data of still images may be registered, for example, or the file names of the image data of still images, for example. The measure field 2316 registers flags (measure flags) indicating whether a terminal device SP or TA has accepted an intention (a "coping intention") to take measures (procedures, care, or steps) to cope with the event information contained in the received communication signals, as will be described later. For example, in this embodiment, the measure field 2316 registers a measure flag "1", which means that the terminal device SP or TA accepts that there is an intention to cope with the invent information contained in the received communication signal (the event information registered in the event field 2312), or a measure flag "0", which means that the terminal device SP or TA does not accept that there is an intention to cope with the event information contained in the received communication signal. It should be noted that the measure flag "0" indicating no acceptance is registered in the measure field 2316 by default. In a case where the detection time at which the predetermined action is detected or the nurse call reception time at which the nurse call is received is contained in the first event notifying communication signal, the detection time or the nurse call reception time may be registered, instead of the reception time.

The inter-device information storage unit 232 stores the inter-device information, which is a notification destination correspondence relationship, a communication address correspondence relationship, and the like in this embodiment. The notification destination correspondence relationship is a correspondence relationship between sensor IDs as transmission sources and terminal IDs as notification destinations (re-notification destinations), indicating notification destinations (re-notification destinations, re-report destinations, or transmission destinations) to which the first event notifying communication signal and the like transmitted from the sensor devices SU are transmitted. The communication address correspondence relationship is a correspondence relationship between the IDs (sensor IDs and terminal IDs) of the respective devices SU, SP, and TA, and the communication addresses thereof. The terminal IDs (terminal identifiers) are the identifiers for specifying the terminal devices SP and TA, and identifying the terminal devices SP and TA.

In this embodiment, both the notification destination correspondence relationship and the communication address correspondence relationship are stored in a table format in the inter-device information storage unit 232. As shown in Fig. 4A, the notification destination correspondence relationship information table AT that registers this notification destination correspondence relationship includes: a transmission source field 2321 that registers the sensor IDs of the sensor devices SU as the transmission sources; and a notification destination field 2322 that registers the terminal IDs of the terminal devices SP and TA as the transmission destinations to which the communication signals sent from the sensor devices SU corresponding to the sensor IDs registered in the transmission source field 2321 are transmitted, for example. Records are provided for the respective sensor IDs (sensor devices SU). As shown in Fig. 4B, for example, a communication address correspondence relationship information table DT that registers the correspondence address correspondence relationship includes: a terminal ID field 2323 that registers the terminal IDs of the terminal devices SP and TA; and a communication address field 2324 that registers the communication addresses of the terminal devices SP and TA corresponding to the terminal IDs registered in the terminal ID field 2323. Records are provided for the respective terminal IDs (for the terminal devices SP and TA).

It should be noted that each of the sensor IDs, the server IDs, and the terminal IDs may be a serial number formed with a predetermined symbol string, for example, or may be a communication address (in this case, the communication address correspondence relationship can be omitted), for example. Further, in the notification destination correspondence relationship, more than one sensor device SU may be associated with one terminal device SP or TA (including multicasting services such as multicast and broadcast).

The SV sensor information storage unit 233 stores the sensor information. In this embodiment, the sensor information is information relating to the sensor devices SU, and is information in which the sensor ID of each sensor device SU and the monitored person's name of each corresponding monitored person Ob are associated with each other.

In this embodiment, such sensor information is stored in a table format in the SV sensor information storage unit 233. More specifically, as shown in Fig. 5, for example, the server-side sensor information table (SV sensor information table) ST-SV that registers the sensor information includes: a sensor ID field 2331 that registers the sensor IDs; an installation location field 2332 that registers the installation locations of the sensor device SU having the sensor IDs registered in the sensor ID field 2331; a monitored person name field 2333 that registers the monitored persons' names of the monitored persons Ob being monitored by the sensor devices SU having the sensor IDs registered in the sensor ID field 2331 (or the monitored persons Ob being at the installation locations of the sensor devices SU having the sensor IDs registered in the sensor ID field 2331); and a remarks field 2334 that registers remarks regarding the sensor devices SU having the sensor IDs registered in the sensor ID field 2331, the installation locations thereof, and the monitored persons Ob. Records are provided for the respective sensor IDs (or the sensor devices SU).

The format information storage unit 234 stores the format information. The format information is information about the record format for inputting details of nursing care including nursing and/or caregiving as a nursing care records, and the record format includes more than one formats that are prepared beforehand as predetermined forms (formats or input forms) in accordance with the types of the nursing care.

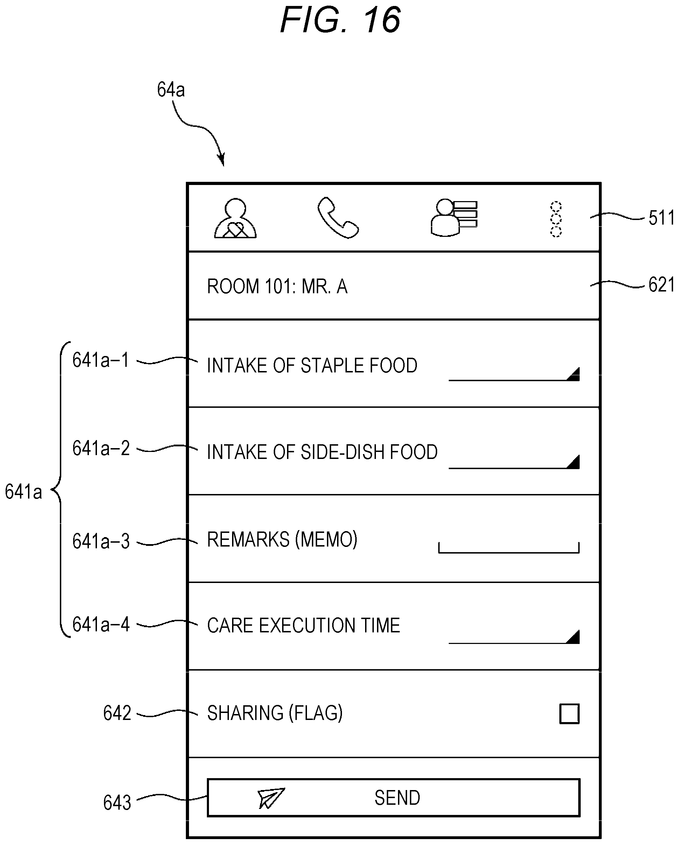

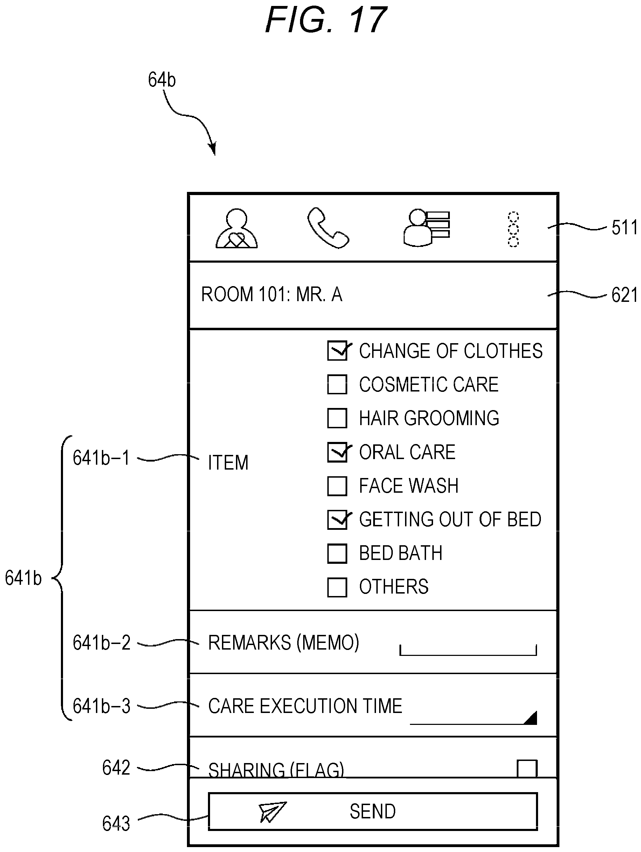

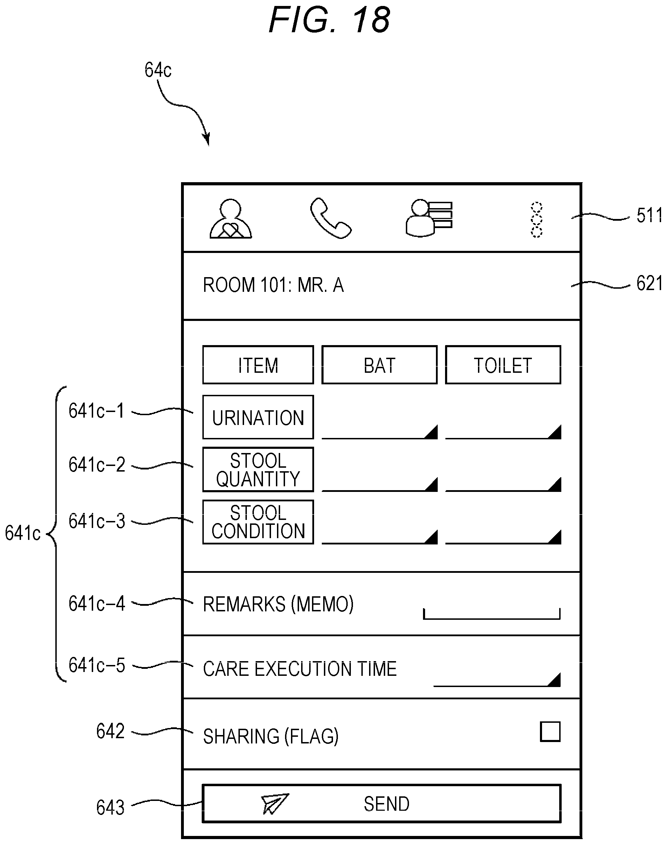

There are various kinds of nursing care, depending on the practice of nursing, the practice of caregiving, and the like. For example, the types of nursing care include regular care, location-dependent care, and the like. More specifically, examples of the regular care include: daily care, such as morning care (changing clothes, face washing, denture attachment, and the like), food care (breakfast care, lunch care, and dinner care), snacking care, administration care (medication care), toothbrushing care, hydration care, vital checks, evening care (changing clothes, toothbrushing, denture detachment, climbing into bed, and the like), doctor's rounds, and changing lying positions; and care to be carried out on a certain day of the week or a certain day of the month, such as bathing care, rehabilitation, recreation, doctor's visits, body cleansing, and body weight measurement. The location-dependent care includes toilet support and the like. These details of nursing care depend on the locations of the living room, the rooms RM, the toilet, the bath room, the nurse station, and the like, or depend on the nurses and the caregivers or the monitoring person in charge of the nursing care or the like, for example. The record formats are prepared beforehand in accordance with the details of such nursing care, and include a record format for morning care, a record format for food care, a record format for snacking care, a record format for medication care (a record format for administration care), a record format for toothbrushing care, a record format for hydration care, and the like, for example. An example thereof is shown in Figs. 16 through 18, which will be described later. In Fig. 16, an example of the record format for food care is shown on a screen displayed on a mobile terminal device TA. In Fig. 17, an example of the record format for morning care is shown on a screen displayed on a mobile terminal device TA. In Fig. 18, an example of the record format for toilet support is shown on a screen displayed on a mobile terminal device TA. In this embodiment, the format information is an electronic file in a record format that forms a record format screen to be displayed on such a terminal device SP or TA. The electronic file in the record format is written in a markup language such as the hyper text markup language (HTML) or the extensible markup language (XML), for example, so that record formats compliant with the predetermined form (format or input form) can be displayed on the predetermined record format screen of the terminal device SP or TA, and is stored in the format information storage unit 234.

The nursing care record information storage unit 235 stores the nursing care record information. The nursing care record information in which the names of the monitored persons as the subjects of nursing care are associated with the details of the nursing care records is stored in the nursing care record information storage unit 235.

The SV control processing unit 22 is a circuit for controlling the respective components of the management server device SV in accordance with the functions of the respective components. When receiving a notification of the predetermined event from a sensor device SU, the SV control processing unit 22 manages the monitoring information concerning the monitoring of the corresponding monitored person Ob, notifies the predetermined terminal device SP or TA of the predetermined event, provides the client with the data in response to a request from the client, and thus, manages the entire monitored-person monitoring system MS. In this embodiment, the SV control processing unit 22 performs predetermined information processing relating to the nursing care records, such as transmission of a predetermined record format or storage of a nursing care record written with the use of the predetermined record format, for example. The SV control processing unit 22 includes a central processing unit (CPU) and peripheral circuits thereof, for example. As the control processing programs are executed, the SV control processing unit 22 functionally includes a server-side control unit (a SV control unit) 221, a server-side monitoring processing unit (a SV monitoring processing unit) 222, and a server-side nursing care record processing unit (a SV nursing care record processing unit) 223.

The SV control unit 221 controls the respective components of the management server device SV in accordance with the functions of the respective components, and performs overall control of the management server device SV.

The SV monitoring processing unit 222 performs predetermined information processing relating to monitoring of the monitored persons Ob. More specifically, when receiving a notification of the predetermined event from a sensor device SU, the SV monitoring processing unit 222 manages the monitoring information concerning the monitoring of the corresponding monitored person Ob, and notifies the predetermined terminal device SP or TA of the predetermined event. More specifically, upon receipt of the first event notifying communication signal from a sensor device SU, the SV monitoring processing unit 222 stores (records) the monitoring information concerning the monitoring of the monitored person Ob contained in the received first event notifying communication signal into the SV monitoring information storage unit 231. The SV monitoring processing unit 222 selects (retrieves), from the notification destination correspondence relationship stored in the inter-device information storage unit 232, the notification destination (a re-notification destination, a report destination, a transfer destination, or a transmission destination) corresponding to the sensor device SU that has transmitted the received first event notifying communication signal, and transmits a second event notifying communication signal to the selected terminal device SP or TA. This selection (retrieval process) is performed in accordance with the sensor ID corresponding to the sensor device SU that has transmitted the received first event notifying communication signal. In a case where the event information contained in the first event notifying communication signal indicates the predetermined action (at least one of the following actions: going to bed, waking up, getting out of bed, and falling), the second event notifying communication signal contains the sensor ID contained in the first event notifying communication signal, the event information and the target image, and a moving image download destination that is the communication address corresponding to the sensor device SU having the sensor ID contained in the first event notifying communication signal. This communication address is selected (retrieved) from the communication address correspondence relationship in accordance with the sensor ID corresponding to the sensor device SU that has transmitted the received first event notifying communication signal. In a case where the event information contained in the first event notifying communication signal is the nurse call, the second event notifying communication signal contains the sensor ID and the event information (nurse call) contained in the first event notifying communication signal.

The SV nursing care record processing unit 223 performs predetermined information processing concerning the nursing care records, which are records of the details of nursing care including nursing and/or caregiving. In this embodiment, the SV nursing care record processing unit 223 performs information processing on a nursing care record using a predetermined record format in two modes: a first mode and a second mode.

In the first mode, only one person is determined as the subject to be subjected to nursing care recording, and one type of record format corresponding to the type of nursing care is selected for the one subject. The details of the nursing care carried out for the one subject are input with the use of the selected one type of record format, and thus, the nursing care recording is performed. In this first mode, in a case where the SV communication IF unit 21 receives a communication signal (a first type notifying communication signal) for sending a notification of the type of nursing care in the nursing care record to be input for one monitored person Ob from a terminal device SP or TA, the SV nursing care record processing unit 223 extracts the record format corresponding to the type of the nursing care contained in the first type notifying communication signal from the format information storage unit 234 of the SV storage unit 23, and returns, through the SV communication IF unit 21, a communication signal (a first format notifying communication signal) containing the extracted record format to the terminal device SP or TA as the transmission source that has transmitted the type notifying communication signal. More specifically, the SV nursing care record processing unit 223 transmits and receives respective communication signals to and from the terminal devices SP and TA as described later. By doing so, the SV nursing care record processing unit 223 sequentially performs the respective processes: a subject selecting process to select only one subject (monitored person Ob) to be subjected to nursing care recording, a format selecting process to select only one type of predetermined record format for the one subject, and a nursing care recording process using the record format.

In the second mode, more than one subject to be subjected to nursing care recording are determined, and one type of record format corresponding to the type of nursing care is selected for these determined subjects. The details of the nursing care carried out for the respective determined subjects are input with the use of the selected one type of record format, and thus, the nursing care recording is performed. In this second mode, in a case where the SV communication IF unit 21 receives a communication signal (a second type notifying communication signal) for sending a notification of the type of nursing care in the nursing care record to be input for the respective monitored persons Ob from a terminal device SP or TA, the SV nursing care record processing unit 223 extracts the record format corresponding to the type of the nursing care contained in the type notifying communication signal from the format information storage unit 234 of the VS storage unit 23, and returns, through the SV communication IF unit 21, a communication signal (a second format notifying communication signal) containing the extracted record format to the terminal device SP or TA as the transmission source that has transmitted the first type notifying communication signal, so that the terminal device SP or TA can input the details of the nursing care for the respective monitored persons Ob while changing the monitored persons Ob. More specifically, the SV nursing care record processing unit 223 transmits and receives respective communication signals to and from the terminal devices SP and TA as described later. By doing so, the SV nursing care record processing unit 223 sequentially performs the respective processes: a subject selecting process to select subjects (monitored persons Ob) to be subjected to nursing care recording, a format selecting process to select only one type of predetermined record format for the subjects, and a nursing care recording process using the record format for each of the subjects.

As will be described later, each of the first and second format notifying communication signals contains the data of a record format screen (an electronic file of the record format screen) as the record format that includes a subject name display region for displaying the name(s) of the monitored person(s), and a nursing care record input region for inputting the details of nursing care as a nursing care record. In the second mode, the subject name display region is also a subject association region for associating a displayed monitored person's name with the nursing care record input region. The SV nursing care record processing unit 223 adds the extracted record format into the second format notifying communication signal, so that the terminal device SP or TA can display subject name display regions corresponding to the respective monitored persons in a changeable manner while displaying the nursing care record input region in a fixed manner.

In the second mode, in a case where the SV nursing care record processing unit 223 has received the respective nursing care records of all the monitored persons Ob, the SV nursing care record processing unit 223 transmits a communication signal (a selectable type notifying communication signal) containing the data of a format selection screen (an electronic file of the format selection screen) for displaying the types of selectable record formats and selecting a record format type, to the terminal device SP or TA as the transmission source that has transmitted the type notifying communication signal.

It should be noted that, as indicated by dashed lines in Fig. 2, if necessary, the management server device SV may further include: a server-side input unit (an SV input unit) 24 that is connected to the SV control processing unit 22 and inputs various kinds of commands and various kinds of data, for example; a server-side output unit (a SV output unit) 25 that outputs the various kinds of commands and the various kinds of data input through the SV input unit 24, and the monitoring information concerning the monitoring of the monitored persons Ob and the like; a server-side interface unit (a SV IF unit) 26 that inputs and outputs data to and from external devices; and the like.

Such a management server device SV can be formed with a computer having a communication function, for example.

The fixed terminal device SP is a device that has a communication function to communicate with the other devices SU, SV, and TA via the network NW, a display function to display predetermined information, an input function to input predetermined instructions and data, and the like, and functions as a user interface (UI) of the monitored-person monitoring system MS by inputting predetermined instructions and data to be given to the management server device SV and the mobile terminal devices TA, displaying the monitoring information obtained by the sensor devices SU, and the like. Such a fixed terminal device SP can be formed with a computer having a communication function, for example. Although the fixed terminal device SP as an example of the terminal device operates in the same manner as a mobile terminal device TA, a mobile terminal device TA that is another example of the terminal device is described in this specification.

A mobile terminal device TA has a communication function to communicate with the other devices SV, SP, and SU via the network NW, a display function to display predetermined information, an input function to input predetermined instructions and data, a call function to conduct voice calls, and the like. The mobile terminal device TA is a device for inputting the predetermined instructions and data (including the data of nursing care records) to be given to the management server device SV and the sensor devices SU, displaying the monitoring information obtained by the sensor devices SU, responding to nurse calls or talking to the other end through voice calls with the sensor devices, and the like. As shown in Figs. 6 and 7, in this embodiment, such a mobile terminal device TA includes a terminal-side communication interface unit (a TA communication IF unit) 31, a terminal-side control processing unit (a TA control processing unit) 32, a terminal-side storage unit (a TA storage unit) 33, a terminal-side sound input/output unit (a TA sound input/output unit) 34, a terminal-side input unit (a TA input unit) 35 (35a, 35b), a terminal-side display unit (a TA display unit) 36, and a terminal-side interface unit (a TA IF unit) 37, for example.

The TA sound input/output unit 34 is a circuit that is connected to the TA control processing unit 32 and acquires and inputs external sound and to the mobile terminal device TA. Under the control of the TA control processing unit 32, the TA sound input/output unit 34 generates and outputs sound in accordance with an electrical signal indicating the sound. The TA sound input/output unit 34 includes a speaker or the like that converts an electrical signal of sound (sound data) into a mechanical vibration signal (an acoustic signal) of sound, a microphone or the like that converts a mechanical vibration signal of sound in an audible range into an electrical signal, and the like, for example. The TA sound input/output unit 34 outputs an electrical signal indicating external sound to the TA control processing unit 32, and converts an electrical signal input from the TA control processing unit 32 into a mechanical vibration signal of sound and outputs the mechanical vibration signal.

The TA input unit 35 is a circuit that is connected to the TA control processing unit 32, and accepts a predetermined operation and inputs the predetermined operation to the mobile terminal device TA, for example. The TA input unit 35 may be formed with input switches to which predetermined functions are assigned, for example. The predetermined operation includes various kinds of operations required in monitoring and the like, such as an operation to input an ID for logging in, an operation to requesting a voice call and terminating the voice call, an operation to request a live moving image and terminating the live moving image, an operation to input an intention ("take measures") of the monitoring person (the user) handling the mobile terminal device TA to take measures (procedures, care, or steps) such as lifesaving, nursing, caregiving, or assisting for the monitored person Ob relating to the reported event, and an operation to input a nursing care record using a predetermined record format, for example. The TA display unit 36 is a circuit that is connected to the TA control processing unit 32, and, under the control of the TA control processing unit 32, displays the details of a predetermined operation input from the TA input unit 35, the monitoring information (such as the predetermined event (the type of a predetermined action detected by the sensor device SU or a nurse call accepted by the sensor device SU), images (a still image and a moving image) of a monitored person Ob, the time of reception of the notification, and the like) relating to the monitoring of the monitored person Ob being monitored by the monitored-person monitoring system MS, the record format for inputting a nursing care record, and the like. The TA display unit 36 is a display device such as a liquid crystal display (LCD) or an organic EL display, for example. In this embodiment, as shown in Fig. 6, the TA input unit 35 includes: a position input device 35a of a resistive film type or an electrostatic capacitance type or the like that forms a touch panel with the TA display unit 36, for example, and detects and inputs an operation position; and an input switch 35b that accepts an input of an instruction to return to another predetermined screen (a second screen) in accordance with the type of the screen (a first screen) displayed on the TA display unit 36, and is provided on the housing of the mobile terminal device TA. On the touch panel, the position input device 35a is provided on the display surface of the TA display unit 36, and at least one input detail candidate that can be input to the TA display unit 36 is displayed. For example, when a user such as a nurse or a caregiver (a monitoring person) touches a display position displaying the details of an input desired to be input, the position is detected by the position input device 35a, and the details displayed at the detected position are input as the details of an input of a user operation to the mobile terminal device TA.

The TA IF unit 37 is a circuit that is connected to the TA control processing unit 32, and, under the control of the TA control processing unit 32, inputs and outputs data to and from external devices. For example, the TA IF unit 37 is an interface circuit that uses the Bluetooth (registered trademark) standard, an interface circuit that performs infrared communication compliant with the IrDA standard or the like, an interface circuit that uses the USB standard, or the like.

Like the SV communication IF unit 21, the TA communication IF unit 31 is a communication circuit that is connected to the TA control processing unit 32, and performs communication under the control of the TA control processing unit 32. The TA communication IF unit 31 includes a communication interface circuit compliant with the IEEE 802.11 standard or the like, for example.

The TA storage unit 33 is a circuit that is connected to the TA control processing unit 32 and stores various kinds of predetermined programs and various kinds of predetermined data, under the control of the TA control processing unit 32. The various kinds of predetermined programs include control processing programs, such as a TA control program for controlling the respective components of the mobile terminal device TA in accordance with the functions of the respective components, a TA monitoring processing program for performing predetermined information processing relating to monitoring of the monitored person Ob, a call processing program for performing a voice call with the sensor device SU by using the TA sound input/output unit 34 or the like, a streaming processing program for receiving a moving image delivered from the sensor device SU and displaying the delivered moving image on the TA display unit 36 by streaming reproduction, and a TA nursing care record processing program for performing predetermined information processing relating to a nursing care record, for example. The various kinds of predetermined data include the data necessary in executing the respective programs, such as the terminal ID of the device, the monitoring information about the monitored person Ob, the sensor information about the sensor device SU, and the screen information to be displayed on the TA display unit 36. The TA storage unit 33 includes a ROM, an EEPROM, and the like, for example. The TA storage unit 33 includes a RAM or the like that serves as a working memory for the TA control processing unit 32 and stores data and the like generated during execution of the predetermined programs.

To store the monitoring information, the sensor information, and the screen information, the TA storage unit 33 functionally includes a terminal-side monitoring information storage unit (a TA monitoring information storage unit) 331, a terminal-side sensor information storage unit (a TA sensor information storage unit) 332, and a screen information storage unit 333, and further functionally includes a data temporary storage unit 334.

The TA monitoring information storage unit 331 stores the monitoring information. In this embodiment, the TA monitoring information storage unit 331 stores the monitoring information, which is the sensor ID contained in a second event notifying communication signal received from the management server device SV, event information (going to bed, waking up, getting out of bed, a fall, or a nurse call in this embodiment), the communication address of the sensor device SU as the image and moving image download destination, the reception time of the second event notifying communication signal, presence/absence of measures to be taken, and the like, which are associated with one another. More specifically, as shown in Fig. 3, the TA monitoring information storage unit 331 stores the monitoring information in a terminal-side monitoring information table (a TA monitoring information table) MT-TA that is similar to the SV monitoring information table MT-SV.

The TA sensor information storage unit 332 stores the sensor information. The TA sensor information storage unit 332 stores the sensor ID, the installation location, the monitored person's name, and remarks, which are associated with one another. More specifically, as shown in Fig. 5, the TA sensor information storage unit 332 stores the sensor information in a terminal-side sensor information table (a TA sensor information table) ST-TA that is similar to the SV sensor information table ST-SV.

The screen information storage unit 333 stores beforehand an electronic file of a standby screen 51 or the like which will be described later, for example, and stores an electronic file of a predetermined record format screen 64 or the like received from the management server device SV. These electronic files are to be used relatively often.

The data temporary storage unit 334 temporarily stores data input by the TA input unit 35 only for the time necessary for the processing.

The TA control processing unit 32 is a circuit for controlling the respective components of the mobile terminal device TA in accordance with the functions of the respective components, receiving and displaying the monitoring information about the monitored person Ob, and responding to a nurse call or talking to the other end. In this embodiment, the TA control processing unit 32 performs a process of inputting a nursing care record using a predetermined record format. The TA control processing unit 32 includes a CPU and peripheral circuits thereof, for example. As the control processing programs are executed, the TA control processing unit 32 functionally includes a terminal-side control unit (a TA control unit) 321, a terminal-side monitoring processing unit (a TA monitoring processing unit) 322, a call processing unit 323, a streaming processing unit 324, and a terminal-side nursing care record processing unit (a TA nursing care record processing unit) 325.

The TA control unit 321 controls the respective components of the mobile terminal device TA in accordance with the functions of the respective components, and performs overall control of the mobile terminal device TA.

The TA monitoring processing unit 322 performs predetermined information processing relating to monitoring of the monitored persons Ob. More specifically, in a case where the TA monitoring processing unit 322 receives the second event notifying communication signal transmitted by the management server device SV in response to the first event notifying communication signal transmitted by the sensor device SU, the TA monitoring processing unit 322 stores (records) the monitoring information about the monitored person Ob into the TA monitoring information storage unit 331, in accordance with the respective pieces of information (the respective sets of data) contained in the received second event notifying communication signal. The TA monitoring processing unit 322 causes the TA display unit 36 to display a screen corresponding to each of the pieces of information contained in the received second event notifying communication signal. Upon receipt of a predetermined input operation from the TA input unit 35, the TA monitoring processing unit 322 performs a predetermined process corresponding to the input operation.

The call processing unit 323 performs a voice call with the sensor device SU, using the TA sound input/output unit 34 or the like. More specifically, the call processing unit 323 uses the TA sound input/output unit 34 or the like, for example, to perform a voice call by Voice over Internet Protocol (VoIP), for example, with the sensor device SU as the transmission source that has transmitted the first event notifying communication signal, which has prompted the transmission of the second event notifying communication signal, to the management server device SV.

The streaming processing unit 324 receives a delivery of a moving image (a live moving image, for example) from the sensor device SU, and causes the TA display unit 36 to display the delivered moving image by streaming reproduction.

The TA nursing care record processing unit 325 performs predetermined information processing concerning nursing care records. More specifically, the TA nursing care record processing unit 325 performs a process of inputting a nursing care record using a predetermined record format in the first and second modes (the first and second modes). In the second mode, on the record format screen 64 displayed on the TA display unit 36, when the TA input unit 35 accepts an instruction to associate the monitored person's name shown in the subject name display region displayed on the TA display unit 36 with the details of the nursing care input to the nursing care record input region displayed on the TA display unit 36 and transmit the associated name and nursing care details to the management server device SV, the TA nursing care record processing unit 325 transmits a communication signal (a nursing care record notifying communication signal) containing the monitored person's name shown in the subject name display region displayed on the TA display unit 36 and the details of the nursing care input to the nursing care record input region displayed on the TA display unit 36 to the management server device SV through the TA communication IF unit 31, and causes the TA display unit 36 to newly display a record format screen that includes the subject name display region showing one of the remaining monitored persons Ob excluding the monitored person Ob of the monitored person's name contained in the nursing care record notifying communication signal, and the nursing care record input region. The remaining monitored persons Ob are newly set as the monitored persons Ob.

Such a mobile terminal device TA can be formed with a portable communication terminal device, such as a so-called tablet computer, a smartphone, or a mobile telephone, for example.

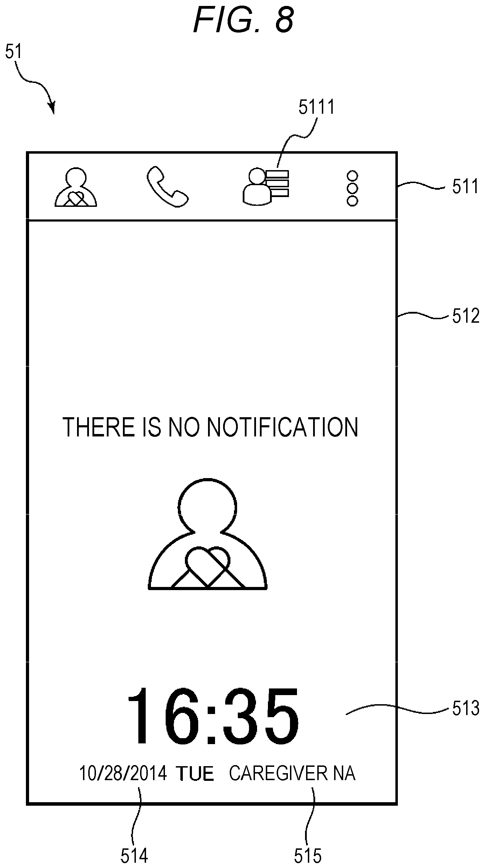

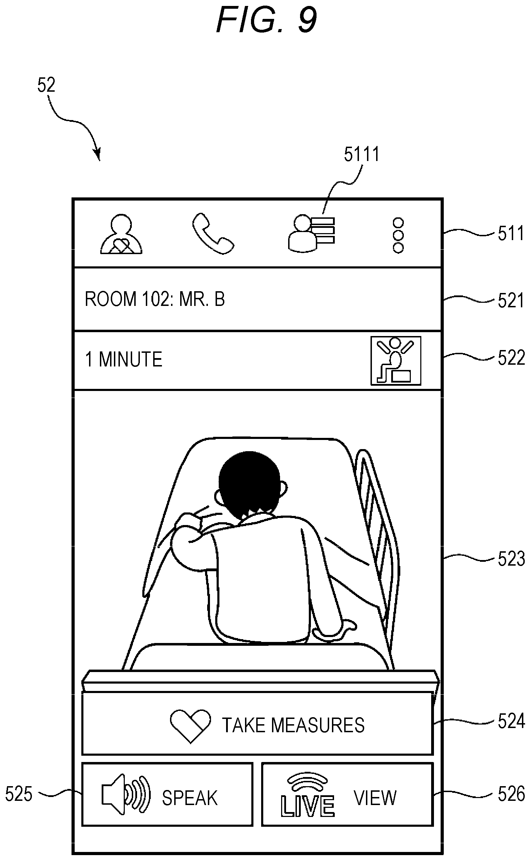

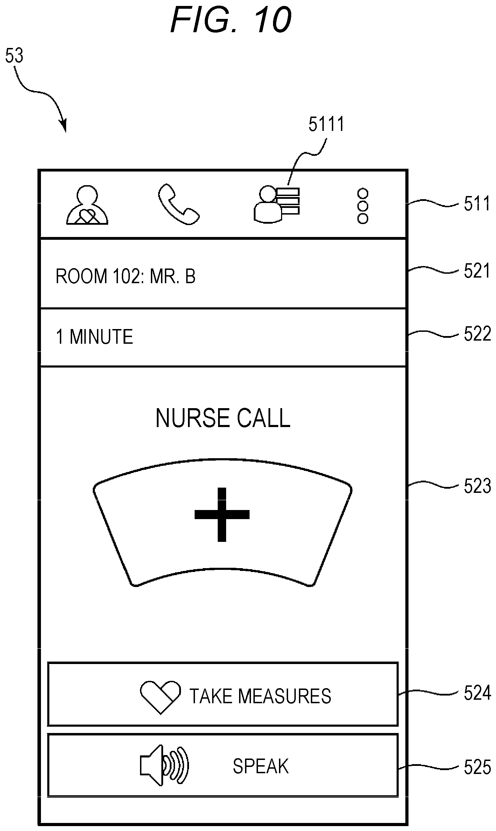

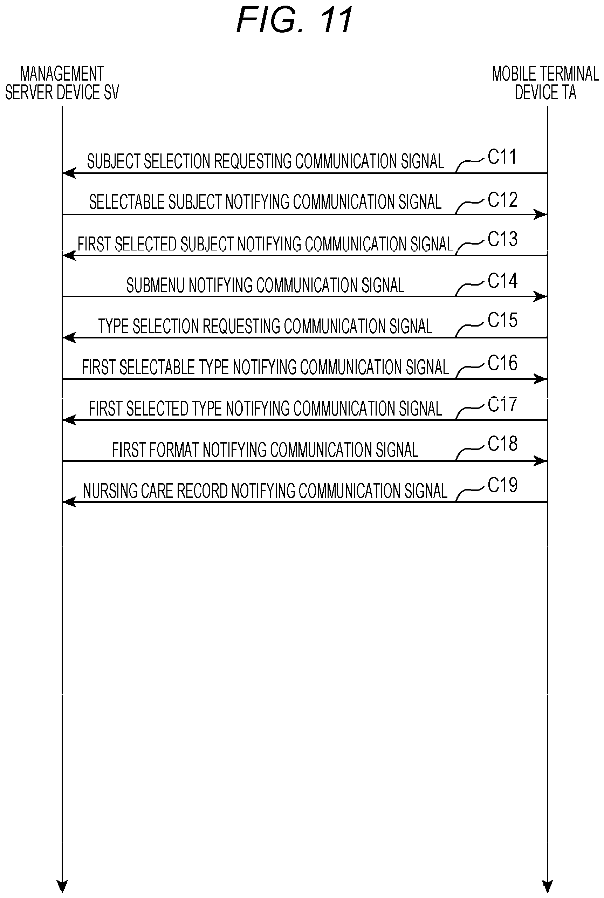

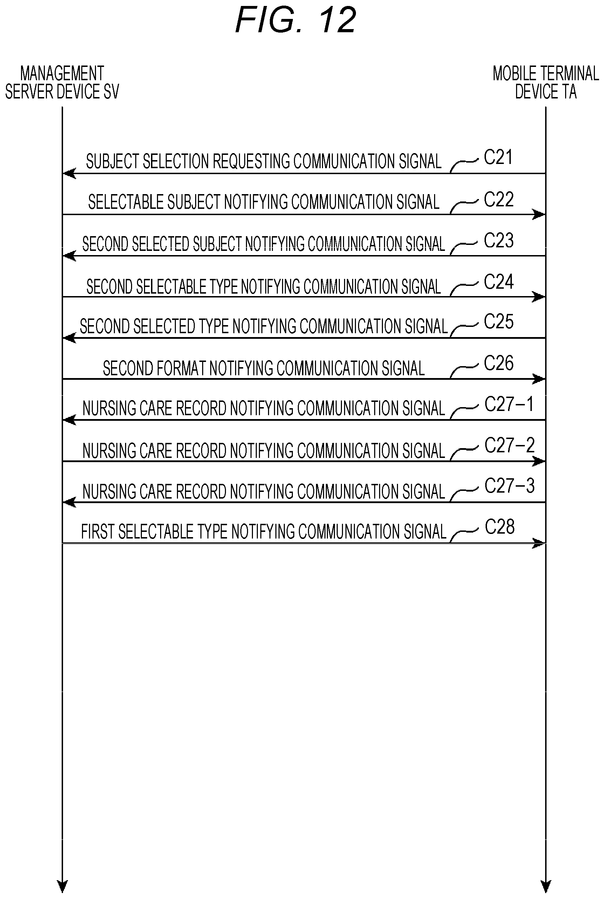

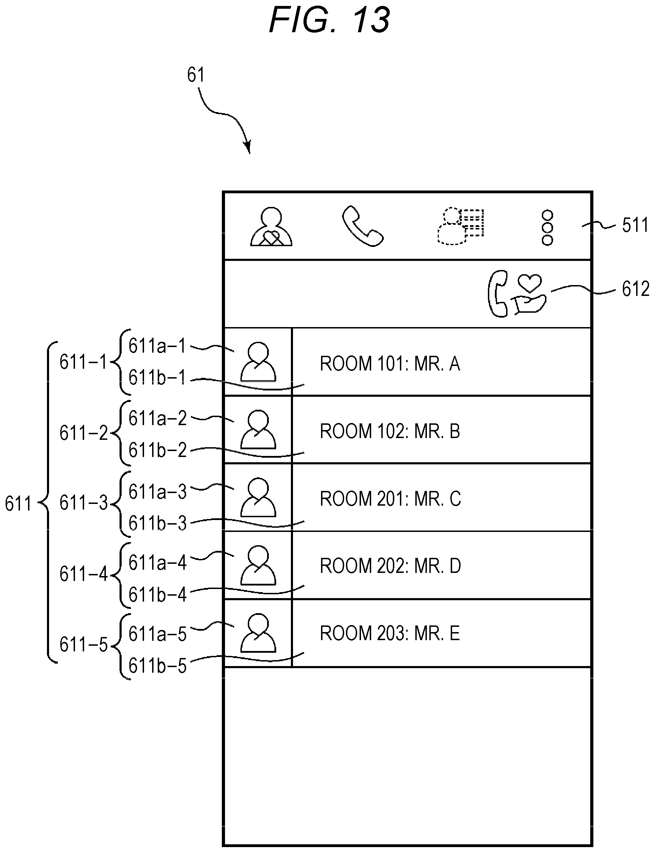

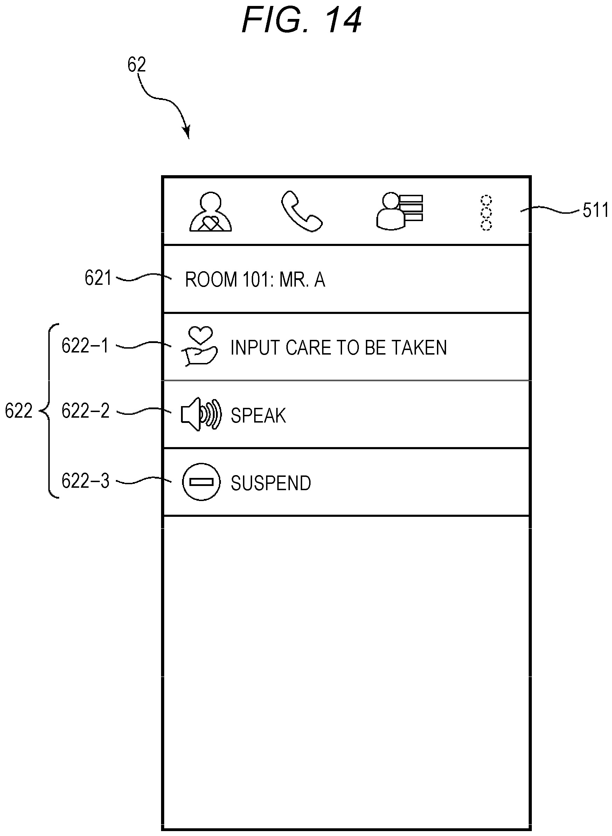

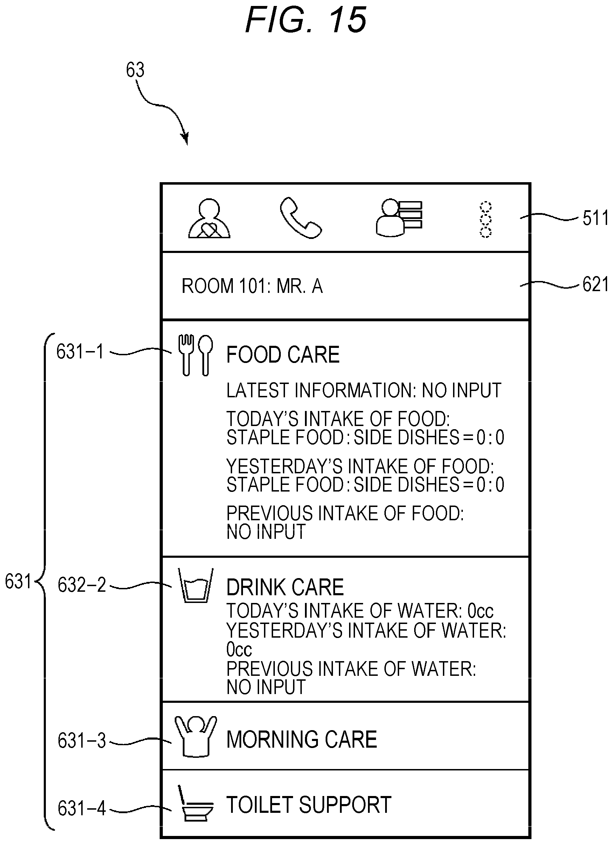

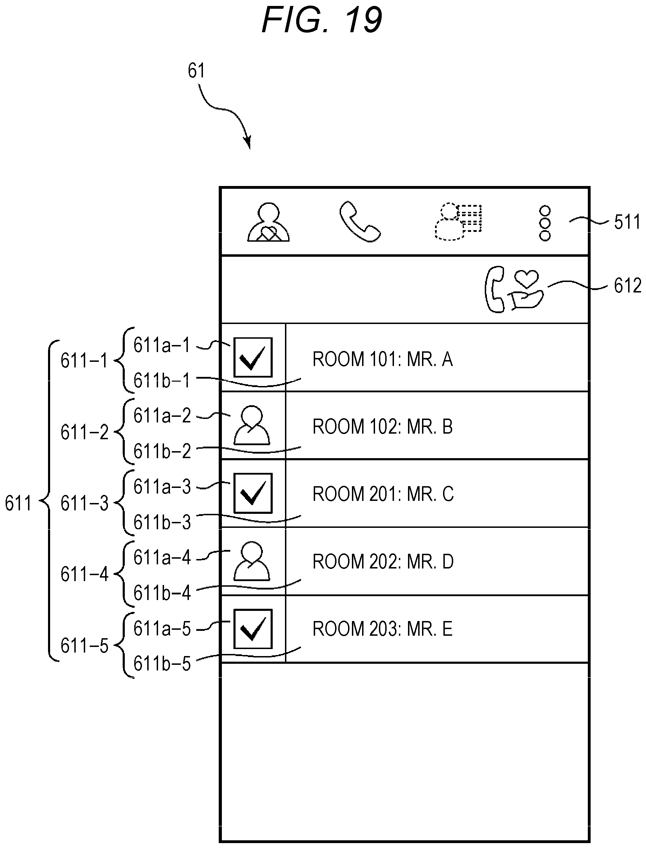

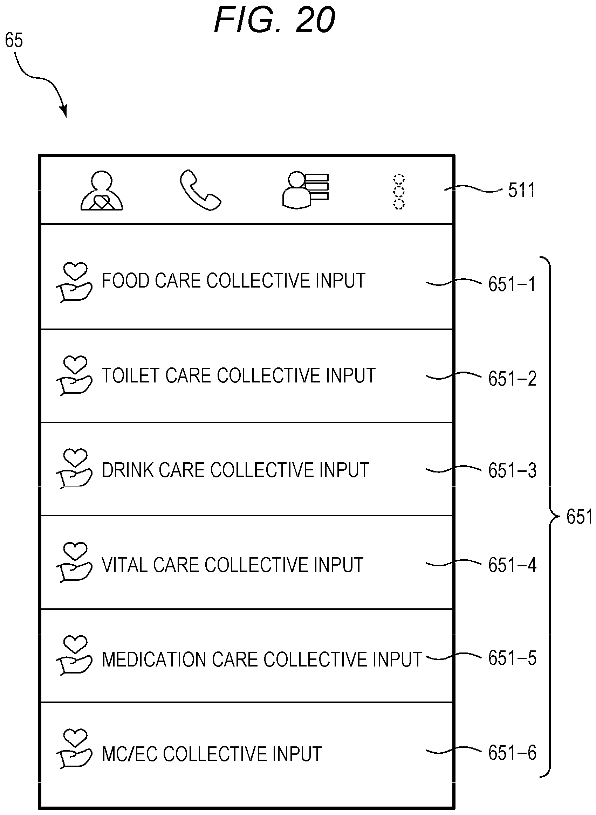

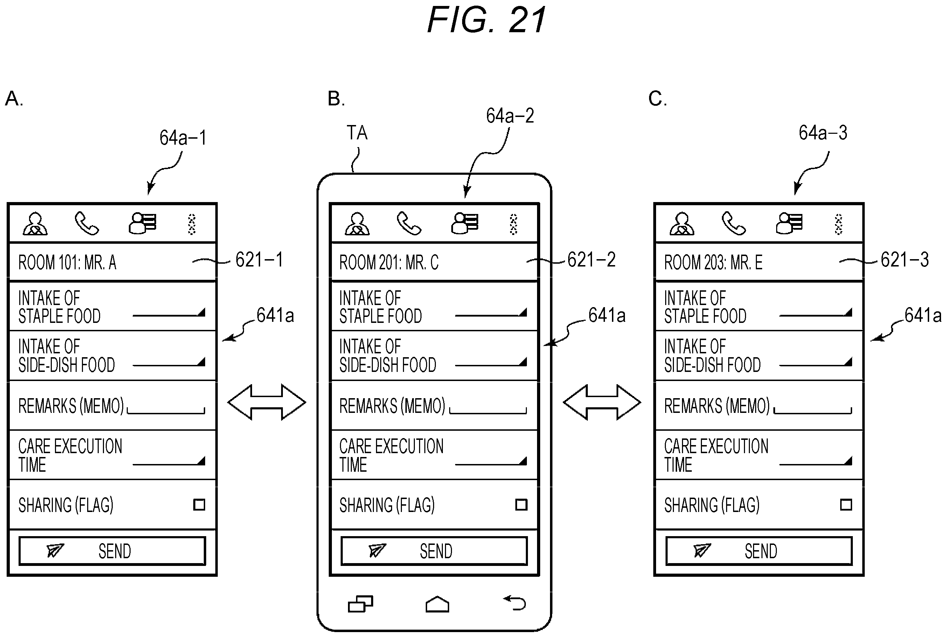

Next, operation of the monitored-person monitoring system according to this embodiment is described. Fig. 8 is a diagram showing an example of a standby screen displayed on the mobile terminal device. Fig. 9 is a diagram showing an example of a monitoring information screen displayed on the mobile terminal device. Fig. 10 is a diagram showing an example of a nurse call receiving screen displayed on the mobile terminal device. Fig. 11 is a sequence diagram showing a nursing care recording operation in the first mode in the monitored-person monitoring system according to the embodiment. Fig. 12 is a sequence diagram showing a nursing care recording operation in the second mode in the monitored-person monitoring system according to the embodiment. Fig. 13 is a diagram showing an example of a subject selection screen displayed on the mobile terminal device. Fig. 14 is a diagram showing an example of a submenu screen displayed on the mobile terminal device. Fig. 15 is a diagram showing an example of a first format selection screen displayed on the mobile terminal device. Fig. 16 is a diagram showing an example of a food care record format screen displayed on the mobile terminal device. Fig. 17 is a diagram showing an example of a morning care record format screen (an evening care record format screen) displayed on the mobile terminal device. Fig. 18 is a diagram showing an example of a toilet support record format screen displayed on the mobile terminal device. Fig. 19 is a diagram showing the subject selection screen after monitored persons are selected on the subject selection screen displayed on the mobile terminal device. Fig. 20 is a diagram showing an example of a second format selection screen displayed on the mobile terminal device. Fig. 21 is a diagram for explaining the procedures for inputting nursing care records in the second mode. Fig. 21A shows the food care record format screen for the first subject in a case where three persons are selected as the subjects in nursing care recording. Fig. 21B shows the food care record format screen for the second subject in the same case.

Fig. 21C shows the food care record format screen for the third subject in the same case. In Fig. 21, the food care record format screen for the second subject shown in Fig. 21B is displayed on the TA display unit 36 of the mobile terminal device TA.

In the description below, a monitoring operation to monitor monitored persons will be first described with reference to Figs. 8 through 10, a nursing care recording process in the first mode will be next described with reference to Fig. 11 and Figs. 13 through 18, and a nursing care recording process in the second mode will be lastly described with reference to Fig. 12, Fig. 13, and Figs. 15 through 21.

In the monitored-person monitoring system MS having the above configuration, each of the devices SU, SV, SP, and TA initializes the respective necessary components and starts its operation once power is applied. In the management server device SV, the SV control unit 221, the SV monitoring processing unit 222, and the SV nursing care record processing unit 223 are functionally formed in the SV control processing unit 21, as the control processing programs are executed. In a mobile terminal device TA, the TA control unit 321, the TA monitoring processing unit 322, the call processing unit 323, the streaming processing unit 324, and the TA nursing care record processing unit 325 are functionally formed in the TA control processing unit 32, as the control processing programs are executed.

### (Monitoring Operation)

In a monitoring operation, a sensor device SU operates as described below for each frame or every several frames, to detect a predetermined action of the monitored person Ob, and determine whether there is a nurse call. First, the sensor device SU acquires a target image that is the image (image data) of one frame from the image sensor, detects a predetermined action of the monitored person Ob in accordance with the acquired target image. When the predetermined action is detected, the sensor device SU transmits the first event notifying communication signal that relates to the detection of the predetermined action and contains the detected predetermined action as the event information to the management server device SV, to notify a predetermined terminal device SP or TA of the detection result. While operating in this manner, the sensor device SU determines whether a nurse call has been received. Upon receipt of a nurse call, the sensor device SU transmits the first event notifying communication signal that relates to the reception of the nurse call and contains the received nurse call as the event information to the management server device SV, to notify the predetermined terminal device SP or TA of the reception of the nurse call.

When the management server device SV receives the first event notifying communication signal from the sensor device SU via the network NW, the SV monitoring processing unit 222 stores (records) the respective pieces of information such as the sensor ID and the event information contained in the first event notifying communication signal as the monitoring information about the monitored person Ob being monitored by the sensor device SU having this sensor ID. The management server device SV then causes the SV monitoring processing unit 222 to acquire, from the notification destination correspondence relationship, the notification destination terminal device SP or TA corresponding to the sensor device SU as the transmission source (notification source) in the received first event notifying communication signal, and transmits the second event notifying communication signal to the notification destination terminal device SP or TA.

When the fixed terminal device SP and the mobile terminal device TA receive the second event notifying communication signal from the management server device SV via the network NW, the TA monitoring processing unit 322 stores (records) the respective pieces of information such as the sensor ID and the event information contained in the second event notifying communication signal as the monitoring information about the monitored person Ob being monitored by the sensor device SU having this sensor ID, and displays the monitoring information.

More specifically, as for a typical operation of a mobile terminal device TA, when power is applied and the operation is started, the mobile terminal device TA receives a login operation performed by the monitoring person (user) such as a nurse or a caregiver, as described above, for example, and the TA monitoring processing unit 322 causes the TA display unit 36 to display the standby screen for awaiting a communication signal directed thereto. As shown in Fig. 8, the standby screen 51 includes a menu bar region 511 that displays a menu bar, a standby main region 512 that displays a message (such as "there is no notification") indicating that the device is in a standby state, a time region 513 that displays the current time, a month/day/year and day-of-the-week region 514 that displays today's date (month/day/year and a day of the week), and a user name region 515 that displays the name of the user who has currently logged into the mobile terminal device TA, for example. When accepting a login operation, the mobile terminal device TA causes the TA monitoring processing unit 322 to transmit a communication signal (a login notifying communication signal) containing the terminal ID of the terminal and the name of the monitoring person (user) who has logged into the device, to the management server device SV. Upon receipt of this login notifying communication signal, the management server device SV causes the SV monitoring processing unit 222 to associate the terminal ID and the name of the monitoring person (monitoring person information) contained in the received login notifying communication signal with each other, and store the terminal ID and the monitoring person's name into the SV storage unit 23.

While waiting for a communication signal directed to the device, the mobile terminal device TA causes the TA control unit 321 of the TA control processing unit 32 to repeatedly determine whether the TA communication IF unit 31 has received a communication signal until a communication signal is received. When a communication signal is received, the TA control unit 321 determines the type of the received communication signal. In a case where the result of this determination shows that the received communication signal is not the second event notifying communication signal, the mobile terminal device TA performs an appropriate process for the received communication signal, ends this process, and then awaits the next communication signal directed thereto. In a case where the received communication signal is the second event notifying communication signal (a second event notification), on the other hand, the mobile terminal device TA causes the TA monitoring processing unit 322 of the TA control processing unit 32 to store (record) the monitoring information about the monitoring of the monitored person Ob contained in the second event notifying communication signal into the TA monitoring information storage unit 331, and cause the TA display unit 36 to display a screen corresponding to each of the pieces of information contained in the received second event notifying communication signal.

More specifically, in a case where the event information contained in the received second event notifying communication signal is the predetermined action, the TA monitoring processing unit 322 causes the TA display unit 36 to display the monitoring information screen 52 shown in Fig. 9, for example. In a case where the event information contained in the received second event notifying communication signal is a nurse call, the TA monitoring processing unit 322 causes the TA display unit 36 to display the nurse call receiving screen 53 shown in Fig. 10, for example.

The monitoring information screen 52 is a screen for displaying the monitoring information concerning the monitoring of the monitored person Ob. As shown in Fig. 9, for example, the monitoring information screen 52 includes: a menu bar region 511; a monitored person name region 521 that displays the installation location of the sensor device SU having the sensor ID contained in the received second event notifying communication signal and the name of the monitored person Ob being monitored by the sensor device SU having the sensor ID; a detected information display region 522 that displays the time elapsed since the time of reception of the received second event notifying communication signal (or the time of detection of the predetermined action) and the event information (the result of detection of the predetermined action) contained in the received second event notifying communication signal; an image region 523 that displays the image contained in the received second event notifying communication signal (in other words, the target image captured by the sensor device SU having the sensor ID) (a still image in this case); a "take measures" button 524; a "speak" button 525; and a "live view" button 526.

To display the installation location of the sensor device SU and the name of the monitored person Ob in the monitored person name region 521, the sensor ID contained in the received second event notifying communication signal is used as the search key, and the installation location of the sensor device SU and the name of the monitored person Ob are retrieved from the TA sensor information storage unit 332, and are then displayed. The detected information display region 522 may display the detection result (going to bed, waking up, getting out of bed, or a fall in this embodiment) contained in the received second event notifying communication signal, but displays an icon that symbolically represents the detection result in this embodiment. To perform display with this icon, the respective actions and icons symbolically representing the respective actions are associated with each other, and are stored beforehand into the TA storage unit 33. In the example shown in Fig. 9, a wake-up icon that symbolically represents waking up is displayed in the detected information display region 522. On the monitoring information screen 52, the "take measures" button 524 is a button for inputting a notification to the mobile terminal device TA to the effect that the user of the mobile terminal device TA has an intention to take predetermined measures (procedures, care, or steps), such as lifesaving, nursing, caregiving, or assisting, for example, in response to the detection result displayed in the monitoring information screen 52. The "speak" button 525 is a button for requesting a voice call, and inputting an instruction to connect the sensor device SU having the sensor ID to the mobile terminal device TA via the network NW so that a call can be made. The "live view" button 526 is a button for requesting a live moving image, and inputting an instruction to display a moving image captured by the sensor device SU having the sensor ID.

The nurse call receiving screen 53 is a screen for displaying reception of a nurse call. As shown in Fig. 10, for example, the nurse call receiving screen 53 includes a menu bar region 511, a monitored person name region 521, a detected information display region 522, a nurse call reception notification display region 531 that displays a message (such as "nurse call") to the effect that a nurse call has been received, a "take measures" button 524, and a "speak" button 525. On the nurse call receiving screen 53, only the time elapsed since the time of reception of the received second event notifying communication signal (or the time of reception of the nurse call) is displayed in the detected information display region 522. It should be noted that the nurse call receiving screen 53 may further include a "live view" button 526.

While the monitoring information screen 52 and the nurse call receiving screen 53 are being displayed, the mobile terminal device TA causes the TA control processing unit 32 to repeat the process until receiving an input operation, and determine whether an input operation has been received through a touch panel formed with the TA input unit 35 and the TA display unit 36. When an input operation is received, the mobile terminal device TA causes the TA control processing unit 32 to perform an appropriate process for the details of the input operation, and ends this process. The mobile terminal device TA then awaits the next communication signal directed thereto.

For example, when an input operation performed on the "take measures" button 524 is received through the TA input unit 35 forming the touch panel, the mobile terminal device TA causes the TA control processing unit 32 to first add information about the reception of an intention of "take measures" to the monitoring information about the monitored person Ob displayed on the TA display unit 36, and stores the information into the TA monitoring information storage unit 331. More specifically, in the TA monitoring information table MT-TA stored in the TA monitoring information storage unit 331, the TA control processing unit 32 registers the measure flag "1" indicating reception of an intention to take measures in the measure field 3316 corresponding to the record storing the monitoring information about the monitored person Ob currently displayed on the TA display unit 36 (in this case, the record storing the monitoring information contained in the received second event notifying communication signal). The TA control processing unit 32 then transmits, to the management server device SV, a communication signal (an intention-to-take-measures notifying communication signal) containing the sensor ID corresponding to the monitoring information about the monitored person Ob displayed on the TA display unit 36, the event information, and information (intention-to-take-measures information) indicating that an intention to "take measures" has been received. The management server device SV, which has received this intention-to-take-measures notifying communication signal, first causes the SV control processing unit 22 to register, in the SV monitoring information table MT-SV stored in the SV monitoring information storage unit 231, the sensor ID and the event information contained in the received intention-to-take-measures notifying communication signal in the sensor ID field 2311 and the event field 2312, respectively, register the measure flag "1" indicating the reception of an intention to take measures in the measure field 2316 in the record storing the measure flag "0" in the measure field 2316, and then transmit a communication signal (an intention-to-take-measures announcing communication signal) containing the sensor ID, the event information, and the intention-to-take-measures information contained in the received intention-to-take-measures notifying communication signal, to the terminal devices SP and TA through a multicasting service. As a result, the respective terminal devices SP and TA share the fact that an intention to "take measures" has been received with respect to the sensor ID corresponding to the monitoring information about the monitored person Ob displayed on the TA display unit 36.

Further, when the TA control processing unit 32 receives an input operation performed on the "speak" button 525, for example, the mobile terminal device TA causes the call processing unit 323 to transmit a communication signal (a call requesting communication signal) containing information indicating a request for a voice call to the sensor device SU monitoring the monitored person Ob displayed on the TA display unit 36, and connect to the sensor device SU responding to the request via the network NW so that a voice call can be made. This enables a voice call between the mobile terminal device TA and the sensor device SU. It should be noted that, when the TA control processing unit 32 receives an input operation performed on an "end" button (not shown) that is a button for inputting an instruction to end a voice call, the mobile terminal device TA causes the call processing unit 323 to transmit a communication signal (a call end communication signal) containing information indicating a request to end a voice call to the sensor device SU monitoring the monitored person Ob displayed on the TA display unit 36. As a result, the voice call between the mobile terminal device TA and the sensor device SU is terminated.

Further, when the TA control processing unit 32 receives an input operation performed on the "live view" button 526, for example, the mobile terminal device TA causes the TA streaming processing unit 324 to transmit a communication signal (a moving image delivery requesting communication signal) containing information indicating a request for a delivery of a live moving image or the like to the sensor device SU monitoring the monitored person Ob currently displayed on the TA display unit 36, connect to the sensor device SU responding to the request via the network NW so that moving images can be downloaded, receive a delivery of a live moving image from the sensor device SU, and cause the TA display unit 36 to display the delivered moving image by streaming reproduction. On the monitoring information screen 52 displaying the live moving image, the moving image is displayed in the image region 523, and a "live end" button (not shown) is displayed in place of the "live view" button 526. As a result, a live moving image is displayed on the mobile terminal device TA. The "live end" button (not shown) is a button for requesting an end of a moving image, and inputting an instruction to end (stop) the delivery of the moving image captured by the sensor device SU having the sensor ID and end (stop) the display. When the TA control processing unit 32 receives an input operation performed on the "live end" button, the mobile terminal device TA causes the TA streaming processing unit 324 to transmit a communication signal (a moving image delivery ending communication signal) containing information such as a request for an end of a moving image delivery or the like to the sensor device SU monitoring the monitored person Ob currently displayed on the TA display unit 36, and causes the TA display unit 36 to display a still image. As a result, the mobile terminal device TA ends the display of the live moving image.

Through such an operation, the monitored-person monitoring system MS monitors each monitored person Ob by roughly detecting predetermined actions in each monitored person Ob and receiving nurse calls, using each sensor device SU, the management server device SV, the fixed terminal device SP, and the mobile terminal devices TA.

### (Nursing Care Recording Operation in the First Mode)

In a nursing care recording process in the first mode, as shown in Fig. 11, a mobile terminal device TA receives, from the TA input unit 35 forming a touch panel, an input operation performed on a subject selection button 5111 in the menu bar region 511 on the standby screen 51, the monitoring information screen 52, the nurse call receiving screen 53, or the like. The mobile terminal device TA then causes the TA nursing care record processing unit 325 to transmit a communication signal (a subject selection requesting communication signal) for requesting selection of a subject to the management server device SV (C11). The subject selection requesting communication signal contains a command (an instruction or a subject selection request command) requesting selection of a subject and the terminal ID of the device. The subject selection button 5111 is a button for inputting an instruction to display a subject selection screen 61 to the mobile terminal device TA.

Upon receipt the subject selection requesting communication signal from the mobile terminal device TA, the management server device SV causes the SV control processing unit 22 to display each monitored person Ob selectable as the subject of the nursing care recording, and return a communication signal (a selectable subject notifying communication signal) containing the data (an electronic file) of the screen (a subject selection screen) for selecting the subject (a monitored person Ob) of the nursing care recording, to the mobile terminal device TA (C12).

As shown in Fig. 13, for example, the subject selection screen 61 includes the menu bar region 511, a subject display region 611 (611-1 through 611-5) that displays one or more selectable subjects in a list, and a "confirm" button 612. The subject display region 611 includes selection check buttons 611a (611a-1 through 611a-5), and subject sub display regions 611b (611b-1 through 611b-5) that are associated with the selection check buttons 611a and are disposed adjacent to the selection check buttons 611a. To display the selectable subjects in a list in the subject display region 611, the SV control processing unit 22 extracts, from the respective records, the monitored persons' names and the installation locations registered in the monitored person name field 3333 and the installation location field 3332, respectively, in the SV sensor information table ST-SV stored in the SV sensor information storage unit 233, and arrange the sets of the installation locations and the monitored persons' names so that the installation locations and the monitored persons' names are sorted sequentially from top to bottom according to a predetermined criterion and are displayed in the subject sub display regions 611b in a case where the TA display unit 36 of the mobile terminal device TA displaying the subject selection screen 61 is viewed from the front side. The sorting criterion may be of any kind, and may be the alphabetical order of the monitored persons' names, the order of the room numbers of the installation locations, or the like, for example. In the example shown in Fig. 13, the sets of the installation locations and the names of the monitored persons are sorted in order of the room numbers of the installation locations. In a case where the subject selection screen 61 is displayed on the TA display unit 36 of the mobile terminal device TA, the subject sub display regions 611b (611b-1 through 611b-5) are used to input one subject (a monitored person Ob), and are formed with buttons (single-subject selection buttons) for inputting the subject selected by the monitoring person (the user of the mobile terminal device TA) to the mobile terminal device TA. In a case where the subject selection screen 61 is displayed on the TA display unit 36 of the mobile terminal device TA, the selection check buttons 611a (611a-1 through 611a-5) are used to input more than one subject (monitored persons Ob), and are formed with buttons (multiple-subject selection buttons) for inputting the subjects selected by the monitoring person (the user of the mobile terminal device TA) to the mobile terminal device TA. The "confirm" button 612 is a button for inputting, to the mobile terminal device TA, an instruction to transmit a communication signal (a selected subject notifying communication signal (first and second selected subject notifying communication signals)) for notifying the management server device SV of the subject(s) input from the subject display region 611, to the management server device SV.

Upon receipt of the selectable subject notifying communication signal from the management server device SV, the mobile terminal device TA causes the TA display unit 36 to display the subject selection screen 61 through the TA control processing unit 32. It should be noted that, in a case where the subject display region 611 is wider than the display region of the TA display unit 36, and not all of the selection check buttons 611a and the subject sub display regions 611b can be displayed in the display region of the TA display unit 36, the portion of the subject display region 611 being displayed in the display region of the TA display unit 36 is changed (scrolled, for example) by a flick input, for example, so that the selection check buttons 611a and the subject sub display regions 611b not displayed in the display region of the TA display unit 36 can be displayed.

In a case where such a subject selection screen 61 is displayed on the TA display unit 36, in the first mode, the user (monitoring person) of the mobile terminal device TA selects one subject by performing an input operation on one of the subject sub display regions 611b and an input operation on the "confirm" button 612.

When accepting the input operation performed on the "confirm" button 612 after accepting the input operation performed on the subject sub display regions 611b from the TA input unit 35 forming the touch panel, the mobile terminal device TA causes the TA control processing unit 32 to transmit, to the management server device SV, a communication signal (the first selected subject notifying communication signal) for notifying the management server device SV of a single subject (a monitored person Ob) input from the subject sub display regions 611b (C13). The first selected subject notifying communication signal contains the monitored person's name of the single subject input through the input operation performed on the subject sub display regions 611b-1 through 611b-5, and the terminal ID of the device.

Upon receipt of the first selected subject notifying communication signal from the mobile terminal device TA, the management server device SV causes the SV control processing unit 22 to associate the monitored person's name contained in the first selected subject notifying communication signal with the terminal ID of the mobile terminal device TA as the transmission source of the first selected subject notifying communication signal, and store the monitored person's name and the terminal ID into the SV storage unit 23. The management server device SV then causes the SV control processing unit 22 to display selectable submenus, and return a communication signal (a submenu notifying communication signal) containing the data (an electronic file) of the screen (submenu screen) for selecting a submenu, to the mobile terminal device TA (C14). As shown in Fig. 14, for example, the submenu screen 62 includes a menu bar region 511, a subject name display region 621 that displays the name of the subject selected on the subject selection screen 61, and a submenu display region 622 (622-1 through 622-3) that displays one or more selectable submenus' names in a list. In the example shown in Fig. 14, the monitoring person operates the mobile terminal device TA to perform an input operation on the region (a subject sub display region, and a single-subject selection button) 611b-1 showing "Room 101, Mr. K. M." on the subject selection screen 61 shown in Fig. 13, and "Room 101: Mr. A" is displayed in the subject name display region 621. In the example shown in Fig. 14, the submenu display region 622 includes submenus that are a "care input" button 622-1, a "speak" button 622-2, and a "suspend" button 622-3. The "care input" button 613 is a button for inputting, to the mobile terminal device TA, an instruction to instruct the monitoring person to record a nursing care record of a subject (a monitored person Ob) into the management server device SV through a terminal device SP or TA. Like the "speak" button 525" described above, the "speak" button 622-2 is a button for requesting a voice call, and inputting an instruction to connect the sensor device SU monitoring the monitored person Ob displayed in the subject name display region 621 to the mobile terminal device TA via the network NW so that a call can be made. The "suspend" button 622-3 is a button for inputting an instruction to temporarily stop the monitoring by the sensor device SU with respect to the monitored person Ob displayed in the subject name display region 621. It should be noted that, in a case where the submenu screen 62 is displayed on the TA display unit 36 of the mobile terminal device TA, the subject name display region 621 may be a button for inputting an instruction to display the subject selection screen 61 to the mobile terminal apparatus TA. Therefore, in this case, the monitoring person can change subjects on the subject selection screen 61 by performing an input operation in the subject name display region 621.

Upon receipt of the submenu notifying communication signal from the management server device SV, the mobile terminal device TA causes the TA display unit 36 to display the submenu screen 62 through the TA control processing unit 32.

In a case where such a submenu screen 62 is displayed on the TA display unit 36, in the first mode, the user (monitoring person) of the mobile terminal device TA performs an input operation on the "care input" button 622-1, to perform nursing care recording for one subject.

When accepting the input operation performed on the "care input" button 622-1, the mobile terminal device TA causes the TA nursing care record processing unit 325 to transmit a communication signal (a type selection requesting communication signal) for requesting selection of a type of nursing care (a type of nursing care recording and a type of record format), to the management server device SV (C15). The type selection requesting communication signal contains a command (an instruction command or a type selection request command) to request selection of a type of nursing care (a type of nursing care recording and a type of record format) and the terminal ID of the device.

Upon receipt of the type selection requesting communication signal from the mobile terminal device TA, the management server device SV causes the SV nursing care record processing unit 223 to return, to the mobile terminal device TA, a communication signal (a first selectable type notifying communication signal) containing the data (an electronic file) of the screen (a first format selection screen) for displaying the types of selectable record formats prepared beforehand in accordance with nursing care types, and selecting one type of record format for one subject (C16).

As shown in Fig. 15, for example, the first format selection screen 63 includes a menu bar region 511, a subject name display region 621 that displays the name of the subject selected on the subject selection screen 61, and a format display region 631 (631-1 through 631-4) that displays one or more selectable record format types and the names thereof in a list for one subject. In a case where the first format selection screen 63 is displayed on the TA display unit 36 of the mobile terminal device TA, the individual regions 632-1 through 631-4 that display the record format names are buttons (first type selection buttons) for inputting the type of a record format (a record format name in the example shown in Fig. 15) selected by the monitoring person, to the mobile terminal device TA. In the example shown in Fig. 15, the format display region 631 includes a "food care" region 631-1 that displays "food care" as a record format name for food care, a "drink care" region 631-2 that displays "drink care" as a record format name for drink care, a "morning care" region 631-3 that displays "morning care" as a record format name for morning care, a "toilet support" region 631-4 that displays "toilet support" as a record format name for toilet support. In a case where the first format selection screen 63 is displayed on the TA display unit 36 of the mobile terminal device TA, the "food care" region 631-1 is a button for inputting selection of a record format for food care to the mobile terminal device TA, the "drink care" region 631-2 is a button for inputting selection of a record format for drink care to the mobile terminal device TA, the "morning care" region 631-3 is a button for inputting selection of a record format for morning care to the mobile terminal device TA, and the "toilet support" region 631-4 is a button for inputting selection of a record format for toilet support to the mobile terminal device TA. Further, in the example shown in Fig. 15, the details of nursing care records are also displayed in the individual regions 631-1 through 631-4 that display the record format names. For example, the details of the nursing care records of today, the previous cay, and the previous time are displayed. To display the details of each of the nursing care records in the region 631, the SV nursing care record processing unit 223 extracts, from the nursing care record information storage unit 235, the details of the nursing care records of today, the previous day, and the previous time corresponding to the subject (the monitored person's name), and generates an electronic file of the first format selection screen 63 by using the extracted details of the nursing care records of today, the previous day, and the previous time.

Upon receipt of the first selectable type notifying communication signal from the management server device SV, the mobile terminal device TA causes the TA display unit 36 to display the first format selection screen 63 through the TA nursing care record processing unit 325. It should be noted that, in a case where the format display region 631 is wider than the display region of the TA display unit 36, and not all of the record format names can be displayed in the display region of the TA display unit 36, the portion of the format display region 631 being displayed in the display region of the TA display unit 36 is changed (scrolled, for example) by a flick input, for example, so that the record format names not displayed in the display region of the TA display unit 36 can be displayed.

When accepting an input operation in the region (first type selection buttons) 631 (631-1 through 631-4) that displays record format names and selection of a record format name from the TA input unit 35 forming the touch panel, the mobile terminal device TA causes the TA nursing care record processing unit 325 to transmit, to the management server device SV, a communication signal (a first selected type notifying communication signal) for issuing a notification of the record format name in the nursing care record to be input for one monitored person (C17). The first selected type notifying communication signal contains the selected record format name as a type of nursing care in the nursing care record to be input for one monitored person, and further contains the terminal ID of the device. The first selected type notifying communication signal is equivalent to an example of the first type notifying communication signal for issuing a notification of the type of nursing care in the nursing care record to be input for one monitored person.

Upon receipt of the first selected type notifying communication signal from the mobile terminal device TA, the management server device SV causes the SV nursing care record processing unit 223 to extract, from the format information storage unit 234, the electronic file of the record format corresponding to the record format name contained in the received first selected type notifying communication signal, and return a communication signal (a first format notifying communication signal) that contains the extracted electronic file of the record format and is designed for issuing a notification of the record format, to the mobile terminal device TA (C18). Upon receipt of the first format notifying communication signal from the management server device SV, the mobile terminal device TA causes the TA display unit 36 to display the record format screen 64 through the TA nursing care record processing unit 325.

For example, when an input operation is performed on the region (the first type selection button) 631 -1 displaying "food care" in process C17, the mobile terminal device TA causes the TA nursing care record processing unit 325 to transmit the first selected type notifying communication signal containing "food care" as the record format name to the management server device SV, receive the first format notifying communication signal containing an electronic file of the record format for food care as a response, and cause the TA display unit 36 to display the food care record format screen 64a shown in Fig. 16, for example.

The food care record format screen 64a is a screen for displaying the record format for food care, and is a screen for inputting the details of conducted food care as a nursing care record. As shown in Fig. 16, for example, the food care record format screen 64a includes: a menu bar region 511; a subject name display region 621 that displays the subject's name; a nursing care record input region 641a for displaying input items to be input as the details of nursing care and inputting the details of the nursing care corresponding to the input items; a sharing check box (□) 642 for inputting a setting as to whether to allow the other terminal devices SP and TA to refer to the nursing care record; and a "send" button 643.

In the record format for food care in this embodiment, the input items include an intake of staple food, an intake of side-dish food, remarks, and a care execution time, for example. To input these items, the nursing care record input region 641a Includes a staple food intake input region 641a-1 for inputting an intake of staple food, a side-dish intake input region 641a-2 for inputting an intake of side-dish food, a free remarks input region 641a-3 for inputting remarks, and a care execution time input region 641a-4 for inputting a time at which nursing care was conducted. The details of nursing care (the details of food care in the example shown in Fig. 16) are input to the nursing care record input region 641a (641a-1 through 641a-4) through an input operation performed on the TA input unit 35 forming the touch panel. In the sharing check box 642, when a check input operation is accepted from the TA input unit 35 forming the touch panel, a check mark is displayed in the sharing check box 642, and an instruction for a setting that allows the other terminal devices SP and TA to refer to the details input to the record format screen 64 (the food care record format screen 64a in the example shown in Fig. 16) is input. If the check mark input operation is not accepted, on the other hand, the sharing check box 642 is left blank, and an instruction for a setting that prohibits the other terminal devices SP and TA from referring to the details input to the record format screen 64 (the food care record format screen 64a in the example shown in Fig. 16) is input. The "send" button 643 is a button for inputting an instruction to transmit the details (the details of food care in the example shown in Fig. 16) input to the record format screen 64 (the food care record format screen 64a in the example shown in Fig. 16) to the management server device SV.

Further, when an input operation is performed on the region (the first type selection button) 631-3 displaying "morning care" in process C17, for example, the mobile terminal device TA causes the TA nursing care record processing unit 325 to transmit the first selected type notifying communication signal containing "morning care" as the record format name to the management server device SV, receive the first format notifying communication signal containing an electronic file of the record format for morning care as a response, and cause the TA display unit 36 to display the morning care record format screen 64b shown in Fig. 17, for example.

The morning care record format screen 64b is a screen for displaying the record format for morning care, and is a screen for inputting the details of conducted morning care as a nursing care record. As shown in Fig. 17, for example, the morning care record format screen 64b includes: a menu bar region 511; a subject name display region 621; a nursing care record input region 641b for displaying input items to be input as the details of nursing care and inputting the details of the nursing care corresponding to the input items; a sharing check box (□) 642; and a "send" button 643.

In the record format for morning care in this embodiment, the input items include changing clothes, cosmetic care, hair grooming, oral care, face washing, getting out of bed, bed bath, and other items, in addition to remarks and a care execution time, for example. To input these items, the nursing care record input region 641b includes: an item region 641b-1 that includes a dressing input check box (□) for inputting presence/absence of a change of clothes, a cosmetic care input check box (□) for inputting presence/absence of cosmetic care, a hair grooming input check box (□) for inputting presence/absence of hair grooming, a mouth care input check box (□) for inputting presence/absence of oral care, a face washing input check box (□) for inputting presence/absence of face washing, an out-of-bed input check box (□) for inputting presence/absence of an act of getting out of bed, a bed bath input check box (□) for inputting presence/absence of bed bath, another input check box (□) for inputting presence/absence of any other item; a free remarks input region 641b-2 for inputting remarks; and a care execution time input region 641b-3 for inputting a time at which nursing care was conducted. When a check mark input operation is accepted from the TA input unit 35 forming the touch panel, a check mark is displayed in a check box, and execution of the nursing care of the input item corresponding to the check box is input.

It should be noted that the morning care record format screen 64b is also a screen for displaying the record format for evening care, and is also used as a screen for inputting the details of conducted evening care as a nursing care record.

Further, when an input operation is performed on the region (the first type selection button) 631-4 displaying "toilet support" in process C17, for example, the mobile terminal device TA causes the TA nursing care record processing unit 325 to transmit the first selected type notifying communication signal containing "toilet support" as the record format name to the management server device SV, receive the first format notifying communication signal containing an electronic file of the record format for toilet support as a response, and cause the TA display unit 36 to display the toilet support record format screen 64c shown in Fig. 18, for example.

The toilet support record format screen 64c is a screen for displaying the record format for toilet support, and is a screen for inputting the details of conducted toilet support as a nursing care record. As shown in Fig. 18, for example, the toilet support record format screen 64c includes: a menu bar region 511; a subject name display region 621; a nursing care record input region 641c for displaying input items to be input as the details of nursing care and inputting the details of the nursing care corresponding to the input items; a sharing check box (□) 642; and a "send" button 643.

In the record format for toilet support in this embodiment, the input items include urination, a stool quantity, a stool condition, remarks, and a care execution time, for example. To input these items, the nursing care record input region 641c Includes a urination input region 641c-1 for inputting presence/absence of urination, a stool quantity input region 641c-2 for inputting a stool quantity, a stool condition input region 641c-3, for inputting a stool condition, a free remarks input region 641c-4 for inputting remarks, and a care execution time input region 641c-5 for inputting a time at which nursing care was conducted. The details of nursing care (the details of toilet support in the example shown in Fig. 18) are input to the nursing care record input region 641c (641c-1 through 641c-5) through an input operation performed on the TA input unit 35 forming the touch panel.

Referring back to Fig. 11, the mobile terminal device TA next causes the TA nursing care record processing unit 325 to transmit, to the management server device SV, a communication signal (a nursing care record notifying communication signal) for transmitting the details of the nursing care input with the use of the record format displayed on the TA display unit 36 as a nursing care record to the management server device SV (C19). After that, the TA nursing care record processing unit 325 causes the TA display unit 36 to display the screen (the standby screen 51, the monitoring information screen 52, the nurse call receiving screen 53, or the like) displaying the subject selection button 5111 accepted in process C11, and then ends this process.

Although not shown in the drawings, a drink care record format screen, a vital care record format screen, a medication care record format screen (an administration care record format screen), and the like are prepared in advance, and are stored in the format information storage unit 234.

More specifically, the monitoring person (user) first refers to the record format displayed on the TA display unit 36, and inputs the details of the nursing care corresponding to the input items in this record format. The input details of the nursing care are associated with the monitored person's name displayed in the subject name display region 621, and are temporarily stored as a nursing care record into the data temporary storage unit 334 by the TA nursing care record processing unit 325. For example, in nursing care recording of food care, the monitoring person (user) refers to the food care record format screen 64a displayed on the TA display unit 36, inputs the details of food care to the respective regions 641a-1 through 641a-4 in the nursing care record input region 641a of the food care record format screen 64a, and performs an input operation on the sharing check box (□) 642 as necessary. The monitoring person (user) then performs an input operation on the "send" button 643.

When accepting the input operation performed on the "send" button 643, the mobile terminal device TA causes the TA nursing care record processing unit 325 to transmit, to the management server device SV, a nursing care record notifying communication signal containing the terminal ID of the device, the subject's name (the monitored person's name), and the details of the nursing care record that have been input through the record format screen 64 and are stored in the data temporary storage unit 334 (in the above example, an intake of staple food, an intake of side-dish food, remarks, a care execution time, and permission/prohibition of sharing, which have been input through the food care record format screen 64a). After the transmission, the TA nursing care record processing unit 325 erases (deletes) the details of the nursing care record stored in the data temporary storage unit 334.

Upon receipt of this nursing care record notifying communication signal, the management server device SV causes the SV nursing care record processing unit 223 to associate the subject's name (the monitored person's name) with the details of the nursing care record (the intake of staple food, the intake of side-dish food, the remarks, the care execution time, and the permission/prohibition of sharing in the above example) contained in the received nursing care record notifying communication signal, and store the subject's name and the details of the nursing care record into the nursing care record information storage unit 235.

In the nursing care recording operation in the first mode, the above processes are performed, and nursing care records are input from the mobile terminal device TA using record formats, and are stored (recorded) into the management server device SV.

### (Nursing Care Recording Operation in the Second Mode)

In a nursing care recording process in the second mode, as shown in Fig. 12, the mobile terminal device TA receives, from the TA input unit 35 forming the touch panel, an input operation performed on the subject selection button 5111 in the menu bar region 511 on the standby screen 51, the monitoring information screen 52, the nurse call receiving screen 53, or the like, as in the above described process C11. The mobile terminal device TA then causes the TA nursing care record processing unit 325 to transmit a subject selection requesting communication signal to the management server device SV (C21).

Upon receipt of the subject selection requesting communication signal from the mobile terminal device TA, the management server device SV causes the SV control processing unit 22 to return a selectable subject notifying communication signal to the mobile terminal device TA as in the above described process C12 (C22).

Upon receipt of the selectable subject notifying communication signal from the management server device SV, the mobile terminal device TA causes the TA display unit 36 to display the subject selection screen 61 shown in Fig. 13, for example, through the TA control processing unit 32.

In a case where such a subject selection screen 61 is displayed on the TA display unit 36, in the second mode, the user (monitoring person) of the mobile terminal device TA sequentially performs input operations on selection check buttons 611a, to select more than one subject. After accepting the input operations performed on the selection check buttons 611a, the mobile terminal device TA causes the TA control processing unit 32 to display check boxes indicating the acceptance of the input operations on the selection check buttons 611a. For example, on the subject selection screen 61 shown in Fig. 13, to select "Room 101: Mr. A" as one of the subjects, the user (monitoring person) of the mobile terminal device TA performs an input operation on the first selection check button 611a-1 located adjacent to the subject sub display region 611b-1 displaying "Room 101: Mr. A". To select "Room 201: Mr. C" as another one of the subjects, the user performs an input operation on the third selection check button 611a-3 located adjacent to the subject sub display region 611b-3 displaying "Room 201: Mr. C". To select "Room 203: Mr. E" as yet another one of the subjects, the user performs an input operation on the fifth selection check button 611a-5 located adjacent to the subject sub display region 611b-5 displaying "Room 203: Mr. E". After accepting these input operations performed on the three selection check buttons 611a-1, 611a-3, and 611a-5, the mobile terminal device TA causes the TA control processing unit 32 to display check boxes indicating the acceptance of these input operations on the three selection check buttons 611a-1, 611a-3, and 611a-5, as shown in Fig. 19. To notify the management server device SV of these selected subjects, the user (monitoring person) of the mobile terminal device TA performs an input operation on the "confirm" button 612 on the subject selection screen 61.

When accepting the input operation performed on the "confirm" button 612 after accepting the input operations performed on the selection check buttons 611a from the TA input unit 35 forming the touch panel, the mobile terminal device TA causes the TA control processing unit 32 to transmit, to the management server device SV, a communication signal (a second selected subject notifying communication signal) for notifying the management server device SV of the subjects (monitored persons Ob) input from the selection check buttons 611a (C23). The second selected subject notifying communication signal contains the monitored persons' names of the subjects input through the input operations performed on the selection check buttons 611a-1 through 611a-5 and the terminal ID of the device.

Upon receipt of the second selected subject notifying communication signal from the mobile terminal device TA, the management server device SV causes the SV control processing unit 22 to associate the monitored persons' names contained in the second selected subject notifying communication signal with the terminal ID of the mobile terminal device TA as the transmission source of the second selected subject notifying communication signal, and store the monitored persons' names and the terminal ID as a server-side nursing care recording subject list (a SV subject list) into the SV storage unit 23. The SV nursing care recording subject list is a list of the monitored persons Ob, who have not received nursing care records, among the monitored persons Ob selected as the subjects in the nursing care recording at the mobile terminal device TA. In the above example, the SV nursing care recording subject list is formed with "Room 101: Mr. A", "Room 201: Mr. C", and "Room 203: Mr. E" in the initial stage. The management server device SV then causes the SV nursing care record processing unit 223 to return, to the mobile terminal device TA, a communication signal (a second selectable type notifying communication signal) containing the data (an electronic file) of the screen (a second format selection screen) for displaying the types of selectable record formats prepared beforehand in accordance with nursing care types, and selecting one record format type for the subjects (C24).

As shown in Fig. 20, for example, the second format selection screen 65 includes a menu bar region 511 and a format display region 651 (651-1 through 651-6) that displays one or more selectable record format types and the names thereof in a list for the subjects. Here, in the example shown in Fig. 20, the format display region 651 on the second format selection screen 65 displays not only the record format names but also "collective input" to clearly indicate that the format display region 651 is designed for more than one person. In a case where the second format selection screen 65 is displayed on the TA display unit 36 of the mobile terminal device TA, the individual regions 651-1 through 651-6 that display the record format names with the "collective input" are buttons (second type selection buttons) for inputting the type of a record format (a record format name in the example shown in Fig. 20) selected by the monitoring person, to the mobile terminal device TA. In the example shown in Fig. 20, the format display region 651 includes: a "food care collective input" region 651-1 that displays "food care collective input" as the name of the record format for food care; a "toilet support collective input" region 651-2 that displays "toilet support collective input" as the name of the record format for toilet support; a "drink care collective input" region 651-3 that displays "drink care collective input" as the name of the record format for drink care; a "vital care collective input" region 651-4 that displays "vital care collective input" as the name the record format for vital checks; a "medication care collective input" region 651-5 that displays "medication care collective input" as the name of the record format for medication care (administration care); and a "MC/EC collective input" region 651-6 that displays "MC/EC collective input" as the name of the record format for morning care or evening care (MC/EC). In a case where the second format selection screen 65 is displayed on the TA display unit 36 of the mobile terminal device TA, the "food care collective input" region 651-1 is a button for inputting selection of the record format for food care to the mobile terminal device TA, the "toilet support collective input" region 651-2 is a button for inputting selection of the record format for toilet support to the mobile terminal device TA, the "drink care collective input" region 651-3 is a button for inputting selection of the record format for drink care to the mobile terminal device TA, the "vital care collective input" region 651-4 is a button for inputting selection of the record format for vital checks to the mobile terminal device TA, the "medication care collective input" region 651-5 is a button for inputting selection of the record format for medication care to the mobile terminal device TA, and the "MC/EC collective input" region 651-6 is a button for inputting selection of the record format for MC/EC to the mobile terminal device TA.

Upon receipt of the second selectable type notifying communication signal from the management server device SV, the mobile terminal device TA causes the TA display unit 36 to display the second format selection screen 65 through the TA nursing care record processing unit 325. It should be noted that, in a case where the format display region 651 is wider than the display region of the TA display unit 36, and not all of the record format names with "collective input" can be displayed in the display region of the TA display unit 36, the portion of the format display region 651 being displayed in the display region of the TA display unit 36 is changed (scrolled, for example) by a flick input, for example, so that the record format names with "collective input" not displayed in the display region of the TA display unit 36 can be displayed.

When accepting an input operation in the region (second type selection buttons) 651 that displays record format names with "collective input" and selection of a record format name from the TA input unit 35 forming the touch panel, the mobile terminal device TA causes the TA nursing care record processing unit 325 to transmit, to the management server device SV, a communication signal (a second selected type notifying communication signal) for issuing a notification of the record format name in the nursing care record to be input for more than one monitored persons (C25). The second selected type notifying communication signal contains the selected record format name as the type of nursing care in the nursing care record to be input for each of the monitored persons, and further contains the terminal ID of the device. The second selected type notifying communication signal is equivalent to an example of the type notifying communication signal (the second type notifying communication signal) for issuing a notification of the type of nursing care in the nursing care record to be input for one monitored person.

Upon receipt of the second selected type notifying communication signal from the mobile terminal device TA, the management server device SV causes the SV nursing care record processing unit 223 to extract, from the format information storage unit 234, the electronic file of the record format corresponding to the record format name contained in the received second selected type notifying communication signal, and return a communication signal (a second format notifying communication signal) that contains the extracted electronic file of the record format and is designed for issuing a notification of the record format, to the mobile terminal device TA, so that the mobile terminal device TA can input the details of the nursing care for each of the monitored persons Ob while changing the monitored persons Ob (C26). More specifically, the SV nursing care record processing unit 223 adds the extracted record format into the second format notifying communication signal, so that the mobile terminal device TA can display the subject name display regions 621 corresponding to the respective monitored persons Ob in a changeable manner while displaying nursing care record input regions 641 in a fixed manner. In this case, the subject name display regions 621 are also subject association regions for associating the displayed monitored persons' names with the nursing care record input regions 641. Upon receipt of the second format notifying communication signal from the management server device SV, the mobile terminal device TA causes the TA nursing care record processing unit 325 to display the record format screen 64 on the TA display unit 36, and store the monitored persons' names contained in the received second format notifying communication signal as a terminal-side nursing care recording subject list (a TA subject list) into the data temporary storage unit 334. The TA nursing care recording subject list is a list of the monitored persons Ob that are displayed in the subject name display regions 621 in a changeable manner. Therefore, the mobile terminal device TA causes the TA nursing care record processing unit 325 to change the subject name display regions 621 within the range of the monitored persons Ob shown (listed) in the TA nursing care recording subject list.

For example, in the above example in which three monitored persons Ob are selected as the subjects in the process C23, when an input operation is performed in the region (second type selection button) 651-1 that displays "food care collective input" in process C25, the mobile terminal device TA causes the TA nursing care record processing unit 325 to transmit the second selected type notifying communication signal containing "food care collective input" as the record format name with "collective input" to the management server device SV, receive the second format notifying communication signal containing an electronic file of the record format for food care as a response so that the mobile terminal device TA can input the details of nursing care for each of the three monitored persons Ob while changing the monitored persons Ob, and cause the TA display unit 36 to display the food care record format screen 64a (64a-1, 64a-2, and 64a-3) shown in Fig. 21, for example.

On the food care record format screen 64a (64a-1, 64a-2, and 64a-3) shown in Fig. 21, the mobile terminal device TA causes the TA display unit 36 to display the subject name display regions 621 (621-1 through 621-3) corresponding to the respective monitored persons Ob in a changeable manner while causing the TA display unit 36 to display the nursing care record input region 641a in a fixed manner, through the TA nursing care record processing unit 325. In the example shown in Fig. 21, the food care record format screen 64a-2 showing "Room 201: Mr. C" in the subject name display region 621-2 is displayed on the TA display unit 36 of the mobile terminal device TA, as shown in Fig. 21B. It should be noted that, in this initial display case, the food care record format screen 64a-1 showing "Room 101: Mr. A" in the subject name display region 621-1 as shown in Fig. 21A may be displayed on the TA display unit 36 of the mobile terminal device TA, and the food care record format screen 64a-3 showing "Room 203: Mr. E" in the subject name display region 621-3 as shown in Fig. 21C may be displayed on the TA display unit 36 of the mobile terminal device TA.

In a case where the monitoring person (user) refers to the record format displayed on the TA display unit 36, and inputs the details of nursing care for the subject "Room 201: Mr. C", the food care record format screen 64a-2 showing "Room 201: Mr. C" in the subject name display region 621-2 as shown in Fig. 21B is displayed on the TA display unit 36 of the mobile terminal device TA, and, in this state, the details of the nursing care corresponding to the input items in this record format are input. The input details of the nursing care are associated with the monitored person's name ("Room 201: Mr. C" in this case) displayed in the subject name display region 621, and are temporarily stored as a nursing care record into the data temporary storage unit 334 by the TA nursing care record processing unit 325. In a case where the food care record format screen 64a showing another monitored person's name ("Room 101: Mr. A" or "Room 203: Mr. E" in this case) in the subject name display region 621 is changed to the food care record format screen 64a showing the current monitored person's name ("Room 201: Mr. C" in this case) in the subject name display region 621, the details of the nursing care temporarily stored in the data temporary storage unit 334 are displayed in the nursing care record input region 641a by the TA nursing care record processing unit 325.

In a case where the monitoring person (user) refers to the record format displayed on the TA display unit 36, and inputs the details of nursing care for the subject "Room 101: Mr. A", on the other hand, an input operation such as flicking from left to right, for example, is performed on the subject name display region 621 showing "Room 201: Mr. C" on the food care record format screen 64a-2 shown in Fig. 21B. When an input operation (flicking from left to right in this case) is performed on the subject name display region 621 showing "Room 201: Mr. C", the mobile terminal device TA causes the TA display unit 36 to display the food care record format screen 64a-1 showing "Room 101: Mr. A" in the subject name display region 621 as shown in Fig. 21A, through the TA nursing care record processing unit 325. In this display state, the monitoring person (user) inputs the details of the nursing care corresponding to the input items in the record format. The input details of the nursing care are associated with the monitored person's name ("Room 101: Mr. A" in this case) displayed in the subject name display region 621, and are temporarily stored as a nursing care record into the data temporary storage unit 334 by the TA nursing care record processing unit 325. In a case where the food care record format screen 64a showing another monitored person's name ("Room 201: Mr. C" or "Room 203: Mr. E" in this case) in the subject name display region 621 is changed to the food care record format screen 64a showing the current monitored person's name ("Room 101: Mr. A" in this case) in the subject name display region 621, the details of the nursing care temporarily stored in the data temporary storage unit 334 are displayed in the nursing care record input region 641a by the TA nursing care record processing unit 325.

Alternatively, in a case where the monitoring person (user) refers to the record format displayed on the TA display unit 36, and inputs the details of nursing care for the subject "Room 203: Mr. E" in the display state shown in Fig. 21B, an input operation such as flicking from right to left, for example, is performed on the subject name display region 621 showing "Room 201: Mr. C" on the food care record format screen 64a-2 shown in Fig. 21B. When an input operation (flicking from right to left in this case) is performed on the subject name display region 621 showing "Room 201: Mr. C", the mobile terminal device TA causes the TA display unit 36 to display the food care record format screen 64a-3 showing "Room 203: Mr. E" in the subject name display region 621 as shown in Fig. 21C, through the TA nursing care record processing unit 325. In this display state, the monitoring person (user) inputs the details of the nursing care corresponding to the input items in the record format. The input details of the nursing care are associated with the monitored person's name ("Room 203: Mr. E" in this case) displayed in the subject name display region 621, and are temporarily stored as a nursing care record into the data temporary storage unit 334 by the TA nursing care record processing unit 325. In a case where the food care record format screen 64a showing another monitored person's name ("Room 201: Mr. C" or "Room 101: Mr. A" in this case) in the subject name display region 621 is changed to the food care record format screen 64a showing the current monitored person's name ("Room 203: Mr. E" in this case) in the subject name display region 621, the details of the nursing care temporarily stored in the data temporary storage unit 334 are displayed in the nursing care record input region 641a by the TA nursing care record processing unit 325.

Because of the above operation, the monitoring person (user) can input the details of the nursing care corresponding to all the input items in the record format for a certain subject person, then input the details of the nursing care corresponding to all the input items in the record format for another subject, and likewise input the details of the nursing care corresponding to all the input items in the record format sequentially for each of the subjects. Alternatively, the monitoring person (user) can input the details of the nursing care corresponding to a certain input item in the record format for all the subjects while sequentially changing the subjects, then input the details of the nursing care corresponding to another input item for all the subjects while sequentially changing the subjects, and likewise input the details of the nursing care for each of the input items for all the subjects while sequentially changing the subjects. Alternatively, the monitoring person (user) can input the details of nursing care while changing subjects and input items.

In a case where the details of nursing care to be input (not all the input items need to be input) are input for the subject having the monitored person name displayed in the subject name display region 621, the monitoring person (user) performs an input operation on the "send" button 643. After accepting the input operation performed on the "send" button 643, the mobile terminal device TA causes the TA nursing care record processing unit 325 to transmit, to the management server device SV, a nursing care record notifying communication signal that contains the terminal ID of the device, the subject's name (the monitored person's name) displayed in the subject name display region 621 on the TA display unit 36, and the details of the nursing care record that are stored in the data temporary storage unit 334 and are associated with the subject's name (the monitored person's name) displayed in the subject name display region 621 on the TA display unit 36 (C27 (C27-1 through C27-3)). After the transmission, the TA nursing care record processing unit 325 erases (deletes) the details of the nursing care record that are stored in the data temporary storage unit 334 and are associated with the monitored person's name contained in the transmitted nursing care record notifying communication signal, deletes the monitored person's name contained in the transmitted nursing care record notifying communication signal from the TA nursing care recording subject list, and causes the TA display unit 36 to newly display a record format screen 64 including the subject name display region 621 that displays one of the monitored persons Ob remaining in the deleted TA nursing care recording subject list, and the nursing care record input regions 641. Accordingly, a record format screen 64 that displays the monitored person's name contained in the transmitted nursing care record notifying communication signal in the subject name display region 621 is not displayed.

Upon receipt of this nursing care record notifying communication signal, the management server device SV causes the SV nursing care record processing unit 223 to associate the subject's name (monitored person's name) and the details of the nursing care record contained in the received nursing care record notifying communication signal with each other, store the subject's name and the details of the nursing care record into the nursing care record information storage unit 235, and delete the subject's name (monitored person's name) from the SV nursing care recording subject list that is stored in the SV storage unit 23 and is associated with the terminal ID contained in the received nursing care record notifying communication signal.

For example, in a case where the food care record format screen 64a-2 displaying "Room 201: Mr. C" in the subject name display region 621-2 as shown in Fig. 21B is displayed on the TA display unit 36, when accepting an input operation performed on the "send" button 643, the mobile terminal device TA causes the TA nursing care record processing unit 325 to transmit, to the management server device SV, a nursing care record notifying communication signal containing the terminal ID of the device, "Room 201: Mr. C", and the details of the nursing care record associated with "Room 201: Mr. C" and stored in the data temporary storage unit 334 (in the above example, an intake of staple food, an intake of side-dish food, remarks, a care execution time, and permission/prohibition of sharing, which have been input through the food care record format screen 64a-2). After the transmission, the TA nursing care record processing unit 325 erases (deletes) the details of the nursing care record that are stored in the data temporary storage unit 334 and are associated with "Room 201: Mr. C" contained in the transmitted nursing care record notifying communication signal, deletes "Room 201: Mr. C" contained in the transmitted nursing care record notifying communication signal from the TA nursing care recording subject list, and causes the TA display unit 36 to newly display a record format screen 64a-1 including the subject name display region 621-1 that displays one of the monitored persons Ob remaining in the deleted TA nursing care recording subject list, or one of the monitored persons "Room 101: Mr. A" and "Room 203: Mr. E" in this example, or "Room 101: Mr. A", for example, and the nursing care record input region 641a. It should be noted that, in this case, a record format screen 64a-3 including the subject name display region 621-3 displaying "Room 203: Mr. E" and the nursing care record input region 641a may be newly displayed on the TA display unit 36. As "Room 201: Mr. C" contained in the transmitted nursing care record notifying communication signal is deleted from the TA nursing care recording subject list, the food care record format screens 64a-1 and 64a-3 can be changed between "Room 101: Mr. A" and "Room 203: Mr. E", which remain in the deleted TA nursing care recording subject list.

Upon receipt of this nursing care record notifying communication signal, the management server device SV causes the SV nursing care record processing unit 223 to associate "Room 201: Mr. C" and the details of the nursing care record (in the above example, an intake of staple food, an intake of side-dish food, remarks, a care execution time, and permission/prohibition of sharing) contained in the received nursing care record notifying communication signal with each other, store "Room 201: Mr. C" and the details of the nursing care record into the nursing care record information storage unit 235, and delete "Room 201: Mr. C" from the SV nursing care recording subject list that is stored in the SV storage unit 23 and is associated with the terminal ID contained in the received nursing care record notifying communication signal.

As for "Room 101: Mr. A", a nursing care record notifying communication signal is transmitted likewise (C27-2), and the monitored person's name is deleted from the TA nursing care recording subject list. In the management server device SV, the nursing care record is associated with the monitored person's name and stored, and the monitored person's is deleted from the SV nursing care recording subject list. As for "Room 203: Mr. E", a nursing care record notifying communication signal is transmitted likewise (C27-3), and the monitored person's name is deleted from the TA nursing care recording subject list. In the management server device SV, the nursing care record is associated with the monitored person's name and stored, and the monitored person's is deleted from the SV nursing care recording subject list.

When any monitored person's name no longer exists in the SV nursing care recording subject list associated with the terminal ID, or when the respective nursing care records of all of the monitored persons Ob have been received, the management server device SV causes the SV nursing care record processing unit 223 to transmit a first selectable type notifying communication signal to the mobile terminal device TA (C28). Upon receipt of the first selectable type notifying communication signal from the management server device SV, the mobile terminal device TA causes the TA display unit 36 to display the first format selection screen 63 shown in Fig. 15, for example, through the TA nursing care record processing unit 325.

In the nursing care recording operation in the second mode, the above processes are performed, and nursing care records are input from the mobile terminal device TA using record formats, and are stored (recorded) into the management server device SV.

As described above, in the monitored-person monitoring system MS, the management server device SV as an example of the central processing device, and the central processing method implemented in the management server device SV, the second format notifying communication signal containing a record format extracted from the format information storage unit 234 is returned to the terminal devices SP and TA, so that the terminal devices SP and TA can input the details of nursing care for each of the monitored persons Ob while changing the monitored persons Ob. Accordingly, the terminal devices SP and TA that have received the second format notifying communication signal can output the record format contained in the second format notifying communication signal, so that the details of nursing care can be input for each of the monitored persons Ob while the monitored persons Ob are changed. For the same type of nursing care, the users (monitoring persons) handling the terminal devices SP and TA use the record format corresponding to the type of the nursing care, and can input the details of the nursing care for each of the monitored persons Ob while changing the monitored persons Ob. For example, a vital check, food care, or the like is normally carried out during substantially the same hour. In a nursing care record for the vital check, a record format for vital checks is used, and the details of the nursing care can be input for each of the subjects while only the subjects for the nursing care record are changed. In a nursing care record for food care, a record format for food care is used, and the details of the nursing care can be input for each of the subjects while only the subjects (monitored persons Ob) for the nursing care record are changed. Accordingly, with the monitored-person monitoring system MS, the management server device SV, and the central processing method, there is no need to repeatedly read a nursed person identification ID and select treatment details for each subject as in the nurse call system disclosed in Patent Literature 1. Alternatively, there is no need to repeatedly select a record format type for each subject in the first mode, and the effort and time required for performing nursing care recording can be further reduced in the second mode.

In the monitored-person monitoring system MS, the management server device SV, and the central processing method, the second format notifying communication signal containing a record format extracted from the format information storage unit 234 is returned to the terminal devices SP and TA, so that the terminal devices SP and TA can display the subject name display regions 621 corresponding to the monitored persons Ob in a changeable manner while displaying the nursing care record input regions 641 in a fixed manner. Accordingly, the terminal devices SP and TA that have received the second format notifying communication signal can output the record format contained in the second format notifying communication signal, so that the terminal devices SP and TA can display the subject name display regions 621 corresponding to the monitored persons Ob in a changeable manner while displaying the nursing care record input regions 641 in a fixed manner. The users (monitoring persons) handling the terminal devices SP and TA can input the details of nursing care for each of the monitored persons Ob while changing the monitored persons Ob, through a single action of changing the subject name display regions 621. Accordingly, the monitored-person monitoring system MS, the management server device SV, and the central processing method can further reduce the effort and time required for performing nursing care recording.

In the monitored-person monitoring system MS, the management server device SV, and the central processing method, in a case where the respective nursing care records of all the monitored persons Ob have been received, the first selectable type notifying communication signal containing the data of the first format selection screen 63 is transmitted to the terminal devices SP and TA. Accordingly, after the respective nursing care records of all the monitored persons Ob have been transmitted, the terminal devices SP and TA can receive the first selectable type notifying communication signal and output the first format selection screen 63. In a case where nursing care recording is performed for another type of nursing care after the respective nursing care records of all the monitored persons Ob have been input to the terminal devices SP and TA and been transmitted to the management server device SV, the users (monitoring persons) handling the terminal devices SP and TA do not need to input an instruction to request an output of the first format selection screen 63 to the terminal devices SP and TA, and can select another type from the first format selection screen 63 that is automatically output. Accordingly, the monitored-person monitoring system MS, the management server device SV, and the central processing method can further reduce the effort and time required for performing nursing care recording.

The terminal devices SP and TA of the monitored-person monitoring system MS according to this embodiment cause the TA display unit 36 to newly display the record format screen 64 that includes the subject name display region 621 displaying the monitored person's name of one of the remaining monitored persons Ob excluding the monitored person Ob whose nursing care record has been transmitted to the management server device SV, and the nursing care record input regions 641. As the remaining monitored persons Ob are regarded as the new monitored persons Ob, the range of the monitored persons Ob that can be next selected becomes narrower every time a nursing care record is transmitted. Thus, the next monitored person Ob can be easily selected, and each monitored person Ob can be prevented from being selected twice.

In the above described embodiment, to enable the terminal devices SP and TA to input the details of nursing care for each of the monitored persons Ob while changing the monitored persons Ob, the SV nursing care record processing unit 223 adds a record format extracted from the format information storage unit 234 into the second format notifying communication signal so that the terminal devices SP and TA can display the subject name display regions 621 corresponding to the respective monitored persons Ob in a changeable manner while displaying the nursing care record input regions 641 in a fixed manner. However, the present invention is not limited to this embodiment. For example, the SV nursing care record processing unit 223 may generate record formats corresponding to the respective monitored persons Ob for each of the monitored persons Ob from a record format extracted from the format information storage unit 234, and add the generated record formats into the second format notifying communication signal. In this case, in the terminal devices SP and TA, the entire record format screens 64 corresponding to the respective monitored persons Ob are changed for each subject in response to an input operation such as flicking, for example, and are displayed on the TA display unit 36. Further, the SV nursing care record processing unit 223 may add a record format extracted from the format information storage unit 234, and a record format generation command into the second format notifying communication signal, for example. The record format generation command is for generating record formats corresponding to the respective monitored persons Ob for each of the monitored persons Ob from the record format extracted from the format information storage unit 234. In this case, in the terminal devices SP and TA, record format screens 64 corresponding to the respective monitored persons Ob are generated in accordance with the record format generation command, and the entire record format screens 64 corresponding to the respective monitored persons Ob are changed for each subject in response to an input operation such as flicking, for example, and are displayed on the TA display unit 36.

As this specification discloses technologies in various modes as described above, the principal technologies among them are summarized below.

A central processing device according to one aspect is a central processing device of a monitored-person monitoring system that monitors a monitored person and includes: a sensor device that is provided for the monitored person as a subject to be monitored, and detects a predetermined event relating to the monitored person; the central processing device that is communicably connected to the sensor device, and manages the event detected by the sensor device and received from the sensor device; and a terminal device that is communicably connected to the central processing device, and receives a notification of the event detected by the sensor device via the central processing device. The central processing device includes: a format information storage unit that stores a record format prepared in accordance with the type of nursing care including nursing and/or caregiving, the record format being designed for inputting the details of the nursing care as a nursing care record; and a nursing care record processing unit that extracts the record format corresponding to the type of the nursing care contained in a type notifying communication signal from the format information storage unit, and returns a format notifying communication signal containing the extracted record format to the terminal device as the transmission source that has transmitted the type notifying communication signal, to enable the terminal device to input the details of nursing care for respective monitored persons while changing the monitored persons, in a case where the type notifying communication signal for issuing a notification of the type of nursing care in a nursing care record to be input for each of the monitored persons has been received from the terminal device. In the above central processing device, the nursing care record processing unit preferably generates record formats corresponding to the respective monitored persons for each of the monitored persons from the extracted record format, and adds the generated record formats into the format notifying communication signal. In the above central processing device, the nursing care record processing unit preferably adds the extracted record format, and a record format generation command into the format notifying communication signal, the record format generation command being for generating record formats corresponding to the respective monitored persons for each of the monitored persons from the extracted record format. In the above central processing device, in a case where a first type notifying communication signal for issuing a notification of the type of nursing care in a nursing care record to be input for one monitored person has been received from the terminal device, the nursing care record processing unit preferably acquires, from the format information storage unit, the record format corresponding to the type of nursing care contained in the first type notifying communication signal, and returns a first format notifying communication signal containing the extracted record format to the terminal device as the transmission source that has transmitted the type notifying communication signal. In a case where a second type notifying communication signal for issuing a notification of the type of nursing care in a nursing care record to be input for each of the monitored persons has been received from the terminal device, the nursing care record processing unit preferably extracts, from the format information storage unit, the record format corresponding to the type of nursing care contained in the type notifying communication signal, and returns a second format notifying communication signal containing the extracted record format to the terminal device as the transmission source that has transmitted the first type notifying communication signal, to enable the terminal device to input the details of nursing care for each of the monitored persons while changing the monitored persons.

Such a central processing device returns a format notifying communication signal containing a record format extracted from the format information storage unit to the terminal device, to enable the terminal device to input the details of nursing care for each of the monitored persons while changing the monitored persons. Accordingly, the terminal device that has received the format notifying communication signal can output the record format contained in the format notifying communication signal, so that the details of nursing care can be input for each of the monitored persons while the monitored persons are changed. For the same type of nursing care, the user (monitoring person) handling the terminal device uses the record format corresponding to the type of the nursing care, and can input the details of the nursing care for each of the monitored persons while changing the monitored persons. For example, a vital check, food care, or the like is normally carried out during substantially the same hour. In a nursing care record for the vital check, a record format for vital checks is used, and the details of the nursing care can be input for each of the subjects while only the subjects for the nursing care record are changed. In a nursing care record for food care, a record format for food care is used, and the details of the nursing care can be input for each of the subjects while only the subjects for the nursing care record are changed. Because of this, the central processing device does not need to repeatedly read a nursed person identification ID and select treatment details for each subject as in the nurse call system disclosed in Patent Literature 1, and thus, can further reduce the effort and time required for performing nursing care recording.

In the central processing device according to another aspect, the record format is the data of a record format screen that includes a subject name display region that displays a monitored person's name, and a nursing care record input region for inputting the details of the nursing care as the nursing care record. The subject name display region is also a subject association region for associating the displayed monitored person's name with the nursing care record input region. The nursing care record processing unit adds the extracted record format into the format notifying communication signal, so that the terminal device can display subject name display regions corresponding to the respective monitored persons in a changeable manner while displaying the nursing care record input region in a fixed manner.

Such a central processing device returns a format notifying communication signal containing the record format extracted from the format information storage unit to the terminal device, so that the terminal device can display the subject name display regions corresponding to the monitored persons in a changeable manner while displaying the nursing care record input region in a fixed manner. Accordingly, the terminal device that has received the format notifying communication signal can output the record format contained in the format notifying communication signal, so that the terminal device can display the subject name display regions corresponding to the respective monitored persons in a changeable manner while displaying the nursing care record input region in a fixed manner. The user (monitoring person) handling the terminal device can input the details of nursing care for each of the monitored persons while changing the monitored persons, through a single action of changing the subject name display region. Thus, the central processing device can further reduce the effort and time required for performing nursing care recording.

In the central processing device according to another aspect, in a case where the nursing care record processing unit has received the respective nursing care records of all the monitored persons, the nursing care record processing unit transmits a selectable type notifying communication signal containing the data of a format selection screen for displaying the types of selectable record formats and selecting a record format type, to the terminal device as the transmission source that has transmitted the type notifying communication signal.

In a case where the respective nursing care records of all the monitored persons have been received, the central processing device transmits (returns) the selectable type notifying communication signal containing the data of the format selection screen to the terminal device. Accordingly, after the respective nursing care records of all the monitored persons have been transmitted, the terminal device can receive the selectable type notifying communication signal and output the format selection screen. In a case where nursing care recording is performed for another type of nursing care after the respective nursing care records of all the monitored persons have been input to the terminal device and been transmitted to the central processing device, the user (monitoring person) handling the terminal device does not need to input an instruction to request an output of the format selection screen to the terminal device, and can select another type from the format selection screen that is automatically output. Thus, the central processing device can further reduce the effort and time required for performing nursing care recording.

A central processing method according to another aspect is a central processing method implemented in a monitored-person monitoring system that monitors a monitored person and includes: a sensor device that is provided for the monitored person as a subject to be monitored, and detects a predetermined event relating to the monitored person; a central processing device that is communicably connected to the sensor device, and manages the event detected by the sensor device and received from the sensor device; and a terminal device that is communicably connected to the central processing device, and receives a notification of the event detected by the sensor device via the central processing device. The central processing method includes: an extraction step of extracting a record format corresponding to the type of nursing care contained in a type notifying communication signal from a format information storage unit that stores the record format that is prepared in accordance with the type of nursing care and is designed for inputting the details of the nursing care as a nursing care record, in a case where the type notifying communication signal for issuing a notification of the type of nursing care in a nursing care record to be input for respective monitored persons has been received from the terminal device; and a response step of returning a format notifying communication signal containing the record format extracted in the extraction step to the terminal device as the transmission source that has transmitted the type notifying communication signal, to enable the terminal device to input the details of nursing care for the respective monitored persons while changing the monitored persons.

By such a central processing method, a format notifying communication signal containing the record format extracted in the extraction step is returned to the terminal device, to enable the terminal device to input the details of nursing care for each of the monitored persons while changing the monitored persons. Accordingly, the terminal device that has received the format notifying communication signal can output the record format contained in the format notifying communication signal, so that the details of nursing care can be input for each of the monitored persons while the monitored persons are changed. For the same type of nursing care, the user (monitoring person) handling the terminal device uses the record format corresponding to the type of the nursing care, and can input the details of the nursing care for each of the monitored persons while changing the monitored persons. Accordingly, by the central processing method, there is no need to repeatedly read a nursed person identification ID and select treatment details for each subject as in the nurse call system disclosed in Patent Literature 1, and thus, the effort and time required for performing nursing care recording can be further reduced.

A monitored-person monitoring system according to another aspect is a monitored-person monitoring system that is designed to monitor a monitored person and includes: a sensor device that is provided for the monitored person who is a subject to be monitored, and detects a predetermined event relating to the monitored person; a central processing device that is communicably connected to the sensor device, and manages the event detected by the sensor device and received from the sensor device; and a terminal device that is communicably connected to the central processing device, and receives a notification of the event detected by the sensor device via the central processing device. The central processing device is one of the above described central processing devices.

Including one of the above described central processing devices, the monitored-person monitoring system can further reduce the effort and time required for performing nursing care recording.

In the monitored-person monitoring system according to another aspect, the terminal device includes: an input unit that accepts an input; a display unit that performs display; and a control unit that performs control. The input unit accepts an instruction to associate the monitored person's name shown in a subject name display region displayed on the display unit with the details of the nursing care input to a nursing care record input region displayed on the display unit and transmit the associated name and nursing care details to the central processing device. In a case where the instruction is accepted by the input unit, the control unit transmits, to the central processing device, a nursing care record notifying communication signal containing the monitored person's name shown in the subject name display region displayed on the display unit and the details of the nursing care input to the nursing care record input region displayed on the display unit, and causes the display unit to newly display a record format screen that includes the subject name display region showing one of the remaining monitored persons excluding the monitored person of the monitored person's name contained in the nursing care record notifying communication signal, and the nursing care record input region. The remaining monitored persons are newly set as the monitored persons.

The terminal device of the monitored-person monitoring system causes the display unit to newly display the record format screen that includes the subject name display region displaying the monitored person's name of one of the remaining monitored persons excluding the monitored person whose nursing care record has been transmitted to the central processing device, and the nursing care record input regions. As the remaining monitored persons are regarded as the new monitored persons, the range of the monitored persons that can be next selected becomes narrower every time a nursing care record is transmitted. Thus, the next monitored person can be easily selected, and each monitored person can be prevented from being selected twice.

This application is based on Japanese Patent Application No. 2016-128580, filed on June 29, 2016, the contents of which are incorporated into this application.

To express the present invention, embodiments of the present invention have been appropriately and fully described with reference to the drawings. However, it should be understood that it is easy for those skilled in the art to change and/or modify the above embodiments. Therefore, as long as variations or modifications made by those skilled in the art are of levels within the scope of the claimed invention, the variations and modifications are construed as included in the claimed invention.

### Industrial Applicability

According to the present invention, it is possible to provide a central processing device and a central processing method for a monitored-person monitoring system, and provide the monitored-person monitoring system.

## Claims

1. A central processing device of a monitored-person monitoring system that monitors a monitored person and includes: a sensor device that is provided for the monitored person as a subject to be monitored, and detects a predetermined event relating to the monitored person; the central processing device that is communicably connected to the sensor device, and manages the event detected by the sensor device and received from the sensor device; and a terminal device that is communicably connected to the central processing device, and receives a notification of the event detected by the sensor device via the central processing device,
the central processing device comprising:
a format information storage unit that stores a record format prepared in accordance with a type of nursing care including at least one of nursing and caregiving, the record format being designed for inputting details of the nursing care as a nursing care record; and
a nursing care record processing unit that extracts the record format corresponding to the type of nursing care contained in a type notifying communication signal from the format information storage unit, and returns a format notifying communication signal containing the extracted record format to the terminal device as a transmission source that has transmitted the type notifying communication signal, to enable the terminal device to input details of nursing care for each of a plurality of monitored persons while changing the monitored persons, in a case where the type notifying communication signal for issuing a notification of the type of nursing care in a nursing care record to be input for each of the monitored persons has been received from the terminal device.

2. The central processing device according to claim 1, wherein
the record format is data of a record format screen that includes a subject name display region that displays a monitored person's name, and a nursing care record input region for inputting the details of the nursing care as the nursing care record,
the subject name display region further serves as a subject association region for associating the displayed monitored person's name with the nursing care record input region, and
the nursing care record processing unit adds the extracted record format into the format notifying communication signal, to enable the terminal device to display a plurality of subject name display regions corresponding to the respective monitored persons in a changeable manner while displaying the nursing care record input region in a fixed manner.

3. The central processing device according to claim 1 or 2, wherein
when the nursing care record processing unit has received respective nursing care records of all the monitored persons, the nursing care record processing unit transmits a selectable type notifying communication signal containing data of a format selection screen for displaying selectable record format types and selecting a record format type, to the terminal device as the transmission source that has transmitted the type notifying communication signal.

4. A central processing method implemented in a monitored-person monitoring system that monitors a monitored person and includes: a sensor device that is provided for the monitored person as a subject to be monitored, and detects a predetermined event relating to the monitored person; a central processing device that is communicably connected to the sensor device, and manages the event detected by the sensor device and received from the sensor device; and a terminal device that is communicably connected to the central processing device, and receives a notification of the event detected by the sensor device via the central processing device,
the central processing method comprising:
an extraction step of extracting a record format corresponding to a type of nursing care contained in a type notifying communication signal from a format information storage unit that stores the record format that is prepared in accordance with the type of nursing care and is designed for inputting details of the nursing care as a nursing care record, in a case where the type notifying communication signal for issuing a notification of the type of nursing care in a nursing care record to be input for each of a plurality of monitored persons has been received from the terminal device; and
a response step of returning a format notifying communication signal containing the record format extracted in the extraction step to the terminal device as the transmission source that has transmitted the type notifying communication signal, to enable the terminal device to input details of nursing care for the respective monitored persons while changing the monitored persons.

5. A monitored-person monitoring system for monitoring a monitored person,
the monitored-person monitoring system comprising:
a sensor device that is provided for the monitored person as a subject to be monitored, and detects a predetermined event relating to the monitored person;
a central processing device that is communicably connected to the sensor device, and manages the event detected by the sensor device and received from the sensor device; and
a terminal device that is communicably connected to the central processing device, and receives a notification of the event detected by the sensor device via the central processing device, wherein
the central processing device is the central processing device of any of claims 1 to 3.

6. The monitored-person monitoring system according to claim 5, wherein
the terminal device includes: an input unit that accepts an input; a display unit that performs display; and a control unit that performs control,
the input unit accepts an instruction to associate a monitored person's name shown in a subject name display region displayed on the display unit with details of nursing care input to a nursing care record input region displayed on the display unit and transmit the associated name and nursing care details to the central processing device, and
when the instruction is accepted by the input unit, the control unit transmits, to the central processing device, a nursing care record notifying communication signal containing the monitored person's name shown in the subject name display region displayed on the display unit and the details of the nursing care input to the nursing care record input region displayed on the display unit, and causes the display unit to newly display a record format screen that includes the subject name display region showing one of the remaining monitored persons excluding the monitored person of the monitored person's name contained in the nursing care record notifying communication signal, and the nursing care record input region, the remaining monitored persons being newly set as the monitored persons.
